# EUROPEAN PATENT APPLICATION

(11) **EP 4 538 293 A1**
(43) Date of publication of application: **16.04.2025**
(21) Application number: 23819133.2
(22) Date of filing: 07.06.2023
(51) Int. Cl.: C07K 16/40, C07K 16/28, C12N 15/13, A61P 35/00, A61K 39/00

(54) **CD39/CD73 BISPECIFIC ANTIGEN BINDING PROTEIN AND USE THEREOF**

(30) Priority: 08.06.2022 CN 202210639601
(71) Applicant: Shanghai Huaota Biopharmaceutical Co., Ltd., Shanghai 201203 (CN); Huabo Biopharm (Shanghai) Co., Ltd., Shanghai 201203 (CN)
(72) Inventor: XU, Jingen, Shanghai 201203 (CN); WANG, Shaojie, Shanghai 201203 (CN); ZHU, Xiangyang, Shanghai 201203 (CN); ZHAN, Yifan, Shanghai 201203 (CN); YU, Haijia, Shanghai 201203 (CN); ZHU, Xi, Shanghai 201203 (CN); ZHANG, Lei, Shanghai 201203 (CN); CHEN, Shi, Shanghai 201203 (CN); CUI, Xiaopei, Shanghai 201203 (CN)
(74) Representative: Weickmann & Weickmann PartmbB
(86) International application number: PCT/CN2023/098744
(87) International publication number: WO 2023/236968

(57) **Abstract**

Provided is an isolated antigen-binding protein, which contains a first binding domain and a second binding domain, wherein the first binding domain is capable of binding to a CD39 protein, and the second binding domain is capable of binding to a CD73 protein. Also provided are a nucleic acid molecule encoding the isolated antigen-binding protein, a vector and a cell containing the nucleic acid molecule, a pharmaceutical composition containing the isolated antigen-binding protein, a method for preparing the isolated antigen-binding protein, and the use of the isolated antigen-binding protein.

## Description

### TECHNICAL FIELD

The present application relates to the field of biomedicine, and specifically relates to a CD39/CD73 bispecific antigen-binding protein and use thereof.

### BACKGROUND

CD39, also known as ectonucleoside triphosphate diphosphohydrolase 1 (ENTPDase1), is an integral membrane protein that hydrolyzes ATP phosphates to produce ADP and AMP. CD39 is expressed in many solid tumor cells, participates in tumor immune escape by inhibiting activation, clonal amplification and homing of tumor-specific T cells, and impairs tumor cell killing performed by effector T lymphocytes. CD39 can directly cause the growth, differentiation, invasion, migration and metastasis of cancer cells and regulate angiogenesis. CD39 is important for both the initiation of angiogenesis and the progression of angiogenesis.

CD73, also known as NT5E, is a 5'-nucleotidase ecto (NT5E) with a molecular weight of about 70 Kd, is anchored to the cell surface by glycophosphatidylinositol (GPI), and is present mainly in the form of a dimer. Under normal physiological conditions, CD73 can be expressed in a variety of tissues or organs, e.g., liver, large intestine, kidney, spleen, lung, ovary, and lymph node.

Functionally, CD39 can bind to and hydrolyze extracellular ATP (eATP) to AMP; and CD73 can hydrolyze AMP to ADO. In an ATP-ADO pathway, CD39 is a rate-limiting enzyme of eATP hydrolysis, and CD73 is a rate-limiting enzyme of AMP hydrolysis. CD39 and CD73 can be used for controlling generation of purine nucleotides and regulate signal transmission of the nucleotides. CD39 can hydrolyze eATP, which can effectively activate the activity of immune cells, and thus cause tumor immune escape. ADO generated from hydrolysis by CD73 can activate immune cells, reduce the immune effect of T cells, and thus cause tumor immune escape.

Therefore, it is urgent to research and develop a drug with CD39/CD73 as the targets to provide a new direction for tumor immunotherapy.

### SUMMARY

The present application provides an isolated antigen-binding protein, having one or more of the following properties: (1) capability of binding to a CD39 protein at a K_{D} value of 1×10⁻⁹ M or lower; (2) capability of binding to a CD73 protein at a K_{D} value of 1×10⁻⁹ M or lower; (3) inhibitory effect on enzymatic activity of a CD39 antigen; (4) inhibitory effect on enzymatic activity of a CD73 antigen; (5) thermal stability; (6) capability of binding to human CD39 and/or cynomolgus CD73; (7) capability of binding to human CD73 and/or cynomolgus CD73; and (8) capability of inhibiting growth of tumor cells.

In one aspect, the present application provides an isolated antigen-binding protein, which includes a first binding domain and a second binding domain, wherein the first binding domain is capable of binding to a CD39 protein, and the second binding domain is capable of binding to a CD73 protein.

In some embodiments, the first binding domain of the isolated antigen-binding protein includes an antibody or an antigen-binding fragment thereof.

In some embodiments, the antibody is selected from the group consisting of a monoclonal antibody, a single-chain antibody, a chimeric antibody, a multispecific antibody, a humanized antibody, and a fully human antibody.

In some embodiments, the antigen-binding fragment is selected from the group consisting of Fab, Fab', F(ab)₂, Fv, F(ab')₂, scFv, VHH, di-scFv and dAb fragments.

In some embodiments, the first binding domain of the isolated antigen-binding protein is VHH.

In some embodiments, the first binding domain of the isolated antigen-binding protein includes at least one CDR in an antibody heavy chain variable region VHH, and the VHH includes an amino acid sequence as set forth in SEQ ID NO: 8.

In some embodiments, the first binding domain of the isolated antigen-binding protein includes CDR3, and the CDR3 includes an amino acid sequence as set forth in SEQ ID NO: 1.

In some embodiments, the first binding domain of the isolated antigen-binding protein includes CDR2, and the CDR2 includes an amino acid sequence as set forth in SEQ ID NO: 2.

In some embodiments, the first binding domain of the isolated antigen-binding protein includes CDR1, and the CDR1 includes an amino acid sequence as set forth in SEQ ID NO: 3.

In some embodiments, the first binding domain of the isolated antigen-binding protein includes FR1, the C-terminus of the FR1 is directly or indirectly linked to the N-terminus of the CDR1, and the FR1 includes an amino acid sequence as set forth in SEQ ID NO: 4.

In some embodiments, the first binding domain of the isolated antigen-binding protein includes FR2, the FR2 is between the CDR1 and the CDR2, and the FR2 includes an amino acid sequence as set forth in SEQ ID NO: 5.

In some embodiments, the first binding domain of the isolated antigen-binding protein includes FR3, the FR3 is between the CDR2 and the CDR3, and the FR3 includes an amino acid sequence as set forth in SEQ ID NO: 6.

In some embodiments, the first binding domain of the isolated antigen-binding protein includes FR4, the N-terminus of the FR4 is directly or indirectly linked to the C-terminus of the CDR3, and the FR4 includes an amino acid sequence as set forth in SEQ ID NO: 7.

In some embodiments, the first binding domain of the isolated antigen-binding protein includes VHH, and the VHH includes an amino acid sequence as set forth in SEQ ID NO: 8.

In some embodiments, the second binding domain of the isolated antigen-binding protein includes an antibody or an antigen-binding fragment thereof.

In some embodiments, the antibody is selected from the group consisting of a monoclonal antibody, a single-chain antibody, a chimeric antibody, a multispecific antibody, a humanized antibody, and a fully human antibody.

In some embodiments, the antigen-binding fragment is selected from the group consisting of Fab, Fab', F(ab)₂, Fv, F(ab')₂, scFv, VHH, di-scFv and dAb fragments.

In some embodiments, the second binding domain of the isolated antigen-binding protein includes at least one CDR in an antibody heavy chain variable region VH, and the VH includes an amino acid sequence as set forth in SEQ ID NO: 16 or SEQ ID NO: 33.

In some embodiments, the second binding domain of the isolated antigen-binding protein includes HCDR3, and the HCDR3 includes an amino acid sequence as set forth in SEQ ID NO: 9 or SEQ ID NO: 27.

In some embodiments, the second binding domain of the isolated antigen-binding protein includes HCDR2, and the HCDR2 includes an amino acid sequence as set forth in SEQ ID NO: 10 or SEQ ID NO: 28.

In some embodiments, the second binding domain of the isolated antigen-binding protein includes HCDR1, and the HCDR1 includes an amino acid sequence as set forth in SEQ ID NO: 11 or SEQ ID NO: 29.

In some embodiments, the second binding domain of the isolated antigen-binding protein includes HCDR1, HCDR2 and HCDR3, and the HCDR1, HCDR2 and HCDR3 include amino acid sequences selected from the group consisting of:
(1) the HCDR1 including an amino acid sequence as set forth in SEQ ID NO: 11, the HCDR2 including an amino acid sequence as set forth in SEQ ID NO: 10, and the HCDR3 including an amino acid sequence as set forth in SEQ ID NO: 9; and
(2) the HCDR1 including an amino acid sequence as set forth in SEQ ID NO: 29, the HCDR2 including an amino acid sequence as set forth in SEQ ID NO: 28, and the HCDR3 including an amino acid sequence as set forth in SEQ ID NO: 27.

In some embodiments, the second binding domain of the isolated antigen-binding protein includes H-FR1, the C-terminus of the H-FR1 is directly or indirectly linked to the N-terminus of the HCDR1, and the H-FR1 includes an amino acid sequence as set forth in SEQ ID NO: 12 or SEQ ID NO: 30.

In some embodiments, the second binding domain of the isolated antigen-binding protein includes H-FR2, the H-FR2 is between the HCDR1 and the HCDR2, and the H-FR2 includes an amino acid sequence as set forth in SEQ ID NO: 13 or SEQ ID NO: 31.

In some embodiments, the second binding domain of the isolated antigen-binding protein includes H-FR3, the H-FR3 is between the HCDR2 and the HCDR3, and the H-FR3 includes an amino acid sequence as set forth in SEQ ID NO: 14 or SEQ ID NO: 32.

In some embodiments, the second binding domain of the isolated antigen-binding protein includes H-FR4, the N-terminus of the H-FR4 is directly or indirectly linked to the C-terminus of the HCDR3, and the H-FR4 includes an amino acid sequence as set forth in SEQ ID NO: 15.

In some embodiments, the second binding domain of the isolated antigen-binding protein includes H-FR1, H-FR2, H-FR3 and H-FR4, and the H-FR1, H-FR2, H-FR3 and H-FR4 include amino acid sequences selected from the group consisting of:
(1) the H-FR1 including an amino acid sequence as set forth in SEQ ID NO: 12, the H-FR2 including an amino acid sequence as set forth in SEQ ID NO: 13, the H-FR3 including an amino acid sequence as set forth in SEQ ID NO: 14, and the H-FR4 including an amino acid sequence as set forth in SEQ ID NO: 15; and
(2) the H-FR1 including an amino acid sequence as set forth in SEQ ID NO: 30, the H-FR2 including an amino acid sequence as set forth in SEQ ID NO: 31, the H-FR3 including an amino acid sequence as set forth in SEQ ID NO: 32, and the H-FR4 including an amino acid sequence as set forth in SEQ ID NO: 15.

In some embodiments, the second binding domain of the isolated antigen-binding protein includes VH, and the VH includes an amino acid sequence as set forth in SEQ ID NO: 16 or SEQ ID NO: 33.

In some embodiments, the second binding domain of the isolated antigen-binding protein includes an antibody heavy chain constant region.

In some embodiments, the antibody heavy chain constant region is derived from an IgG heavy chain constant region.

In some embodiments, the antibody heavy chain constant region is derived from a human IgG heavy chain constant region.

In some embodiments, the antibody heavy chain constant region is derived from a human IgG1 heavy chain constant region or a human IgG4 heavy chain constant region.

In some embodiments, the antibody heavy chain constant region includes an amino acid sequence as set forth in SEQ ID NO: 43.

In some embodiments, the second binding domain of the isolated antigen-binding protein includes an antibody heavy chain, and the antibody heavy chain includes an amino acid sequence as set forth in SEQ ID NO: 25 or SEQ ID NO: 41.

In some embodiments, the second binding domain of the isolated antigen-binding protein includes at least one CDR in an antibody light chain variable region VL, and the VL includes an amino acid sequence as set forth in SEQ ID NO: 24 or SEQ ID NO: 40.

In some embodiments, the second binding domain of the isolated antigen-binding protein includes LCDR3, and the LCDR3 includes an amino acid sequence as set forth in SEQ ID NO: 17 or SEQ ID NO: 34.

In some embodiments, the second binding domain of the isolated antigen-binding protein includes LCDR2, and the LCDR2 includes an amino acid sequence as set forth in SEQ ID NO: 18 (WAS) or SEQ ID NO: 35 (LAS).

In some embodiments, the second binding domain of the isolated antigen-binding protein includes LCDR1, and the LCDR1 includes an amino acid sequence as set forth in SEQ ID NO: 19 or SEQ ID NO: 36.

In some embodiments, the second binding domain of the isolated antigen-binding protein includes LCDR1, LCDR2 and LCDR3, and the LCDR1, LCDR2 and LCDR3 include amino acid sequences selected from the group consisting of:
(1) the LCDR1 including an amino acid sequence as set forth in SEQ ID NO: 19, the LCDR2 including an amino acid sequence as set forth in SEQ ID NO: 18 (WAS), and the LCDR3 including an amino acid sequence as set forth in SEQ ID NO: 17; and
(2) the LCDR1 including an amino acid sequence as set forth in SEQ ID NO: 36, the LCDR2 including an amino acid sequence as set forth in SEQ ID NO: 35 (LAS), and the LCDR3 including an amino acid sequence as set forth in SEQ ID NO: 34.

In some embodiments, the second binding domain of the isolated antigen-binding protein includes L-FR1, the C-terminus of the L-FR1 is directly or indirectly linked to the N-terminus of the L-CDR1, and the L-FR1 includes an amino acid sequence as set forth in SEQ ID NO: 20 or SEQ ID NO: 37.

In some embodiments, the second binding domain of the isolated antigen-binding protein includes L-FR2, the L-FR2 is between the LCDR1 and the LCDR2, and the L-FR2 includes an amino acid sequence as set forth in SEQ ID NO: 21 or SEQ ID NO: 38.

In some embodiments, the second binding domain of the isolated antigen-binding protein includes L-FR3, the L-FR3 is between the LCDR2 and the LCDR3, and the L-FR3 includes an amino acid sequence as set forth in SEQ ID NO: 22 or SEQ ID NO: 39.

In some embodiments, the second binding domain of the isolated antigen-binding protein includes L-FR4, the N-terminus of the L-FR4 is directly or indirectly linked to the C-terminus of the LCDR3, and the L-FR4 includes an amino acid sequence as set forth in SEQ ID NO: 23.

In some embodiments, the second binding domain of the isolated antigen-binding protein includes L-FR1, L-FR2, L-FR3 and L-FR4, and the L-FR1, L-FR2, L-FR3 and L-FR4 include amino acid sequences selected from the group consisting of:
(1) the L-FR1 including an amino acid sequence as set forth in SEQ ID NO: 20, the L-FR2 including an amino acid sequence as set forth in SEQ ID NO: 21, the L-FR3 including an amino acid sequence as set forth in SEQ ID NO: 22, and the L-FR4 including an amino acid sequence as set forth in SEQ ID NO: 23; and
(2) the L-FR1 including an amino acid sequence as set forth in SEQ ID NO: 37, the L-FR2 including an amino acid sequence as set forth in SEQ ID NO: 39, the L-FR3 including an amino acid sequence as set forth in SEQ ID NO: 39, and the L-FR4 including an amino acid sequence as set forth in SEQ ID NO: 23.

In some embodiments, the second binding domain of the isolated antigen-binding protein includes VL, and the VL includes an amino acid sequence as set forth in SEQ ID NO: 24 or SEQ ID NO: 40.

In some embodiments, the second binding domain of the isolated antigen-binding protein includes an antibody light chain constant region.

In some embodiments, the antibody light chain constant region is derived from an Igκ constant region.

In some embodiments, the antibody light chain constant region is derived from a human Igκ constant region.

In some embodiments, the antibody light chain constant region includes an amino acid sequence as set forth in SEQ ID NO: 44.

In some embodiments, the second binding domain of the isolated antigen-binding protein includes an antibody light chain, and the antibody light chain includes an amino acid sequence as set forth in SEQ ID NO: 26 or SEQ ID NO: 42.

In some embodiments, the second binding domain of the isolated antigen-binding protein includes VH and VL, and the VH and VL include amino acid sequences selected from the group consisting of:
(1) the VH including an amino acid sequence as set forth in SEQ ID NO: 16, and the VL including an amino acid sequence as set forth in SEQ ID NO: 24; and
(2) the VH including an amino acid sequence as set forth in SEQ ID NO: 33, and the VL including an amino acid sequence as set forth in SEQ ID NO: 40.

In some embodiments, the second binding domain of the isolated antigen-binding protein includes an antibody heavy chain and an antibody light chain, and the antibody heavy chain and the antibody light chain include amino acid sequences selected from the group consisting of:
(1) the antibody heavy chain including an amino acid sequence as set forth in SEQ ID NO: 25, and the antibody light chain including an amino acid sequence as set forth in SEQ ID NO: 26; and
(2) the antibody heavy chain including an amino acid sequence as set forth in SEQ ID NO: 41, and the antibody light chain including an amino acid sequence as set forth in SEQ ID NO: 42.

In some embodiments, the first binding domain and the second binding domain of the isolated antigen-binding protein are directly or indirectly linked.

In some embodiments, the C-terminus of the first binding domain of the isolated antigen-binding protein is linked to the N-terminus of the second binding domain.

In some embodiments, the C-terminus of the first binding domain of the isolated antigen-binding protein is linked to the N-terminus of a heavy chain variable region of the second binding domain.

In some embodiments, the first binding domain of the isolated antigen-binding protein is linked to the second binding domain by a linker.

In some embodiments, the linker includes a peptide linker.

In some embodiments, the linker includes an amino acid sequence of (GGGGS) n, where n is any positive integer from 0 to 10.

In some embodiments, the linker includes an amino acid sequence as set forth in SEQ ID NO: 47.

In some embodiments, the isolated antigen-binding protein includes two of the first binding domains.

In some embodiments, the two first binding domains of the isolated antigen-binding protein are directly or indirectly linked to the N-terminus of the second binding domain respectively.

In some embodiments, the two first binding domains of the isolated antigen-binding protein are directly or indirectly linked to the N-terminus of two heavy chains of the second binding domain respectively.

In some embodiments, the isolated antigen-binding protein includes a first polypeptide chain and a second polypeptide chain, wherein the first polypeptide chain includes heavy chains of the first binding domain and the second binding domain.

In some embodiments, the N-termini of the heavy chains of the first binding domain and the second binding domain of the isolated antigen-binding protein are directly or indirectly connected.

In some embodiments, the N-termini of the heavy chains of the first binding domain and the second binding domain of the isolated antigen-binding protein are connected by a linker.

In some embodiments, the first polypeptide chain of the isolated antigen-binding protein includes an amino acid sequence as set forth in SEQ ID NO: 45 or SEQ ID NO: 46.

In some embodiments, the second polypeptide chain of the isolated antigen-binding protein includes a light chain of the second binding domain.

In some embodiments, the second polypeptide chain of the isolated antigen-binding protein includes an amino acid sequence as set forth in SEQ ID NO: 26 or SEQ ID NO: 42.

In some embodiments, the isolated antigen-binding protein includes two of the first polypeptide chains and two of the second polypeptide chains.

In another aspect, the present application further provides a pharmaceutical combination, including an antigen-binding protein capable of binding to CD39 and an antigen-binding protein capable of binding to CD73.

In some embodiments, the antigen-binding protein capable of binding to CD39 has one or more of the following properties:
(1) capability of binding to human CD39 at an EC50 less than about 0.5 µg/mL according to an FACS detection method;
(2) capability of binding to cynomolgus (cyno) CD39 at an EC50 less than about 0.7 µg/mL according to an FACS detection method;
(3) capability of binding to human CD39 at a KD less than about 1.3×10⁻⁸ M according to a Biacore detection method;
(4) capability of binding to cynomolgus (cyno) CD39 at a KD less than about 1.1×10⁻⁷ M according to a Biacore detection method;
(5) capability of inhibiting ATPase activity in CD39 expressing cells at an EC50 less than about 0.8 µg/mL according to an ATPase activity analysis; and
(6) capability of inhibiting the function of CD39 of tumor cells.

In some embodiments, the antigen-binding protein capable of binding to CD39 includes at least one CDR in a heavy chain variable region VH, and the VH includes an amino acid sequence as set forth in SEQ ID NO: 8.

In some embodiments, the antigen-binding protein capable of binding to CD39 includes HCDR3, and the HCDR3 includes an amino acid sequence as set forth in SEQ ID NO: 1.

In some embodiments, the antigen-binding protein capable of binding to CD39 includes HCDR2, and the HCDR2 includes an amino acid sequence as set forth in SEQ ID NO: 2.

In some embodiments, the antigen-binding protein capable of binding to CD39 includes HCDR1, and the HCDR1 includes an amino acid sequence as set forth in SEQ ID NO: 3.

In some embodiments, the antigen-binding protein capable of binding to CD39 includes VH, and the VH includes an amino acid sequence as set forth in SEQ ID NO: 8.

In some embodiments, the antigen-binding protein capable of binding to CD39 is VHH.

In some embodiments, the antigen-binding protein capable of binding to CD73 has one or more of the following properties:
(1) capability of specifically binding to CD73 or a functional active fragment thereof;
(2) capability of inhibiting the enzymatic activity of CD73;
(3) capability of inducing endocytosis of CD73 on the cell surface; and
(4) capability of inhibiting growth and/or proliferation of tumor cells.

In some embodiments, the antigen-binding protein capable of binding to CD73 includes at least one CDR in an antibody heavy chain variable region VH, and the VH includes an amino acid sequence as set forth in SEQ ID NO: 16 or SEQ ID NO: 33.

In some embodiments, the antigen-binding protein capable of binding to CD73 includes HCDR3, and the HCDR3 includes an amino acid sequence as set forth in SEQ ID NO: 9 or SEQ ID NO: 27.

In some embodiments, the antigen-binding protein capable of binding to CD73 includes HCDR2, and the HCDR2 includes an amino acid sequence as set forth in SEQ ID NO: 10 or SEQ ID NO: 28.

In some embodiments, the antigen-binding protein capable of binding to CD73 includes HCDR1, and the HCDR1 includes an amino acid sequence as set forth in SEQ ID NO: 11 or SEQ ID NO: 29.

In some embodiments, the antigen-binding protein capable of binding to CD73 includes VH, and the VH includes an amino acid sequence as set forth in SEQ ID NO: 16 or SEQ ID NO: 33.

In some embodiments, the antigen-binding protein capable of binding to CD73 includes an antibody heavy chain constant region.

In some embodiments, the antibody heavy chain constant region is derived from an IgG heavy chain constant region.

In some embodiments, the antibody heavy chain constant region is derived from a human IgG heavy chain constant region.

In some embodiments, the antibody heavy chain constant region is derived from a human IgG1 heavy chain constant region or a human IgG4 heavy chain constant region.

In some embodiments, the antibody heavy chain constant region includes an amino acid sequence as set forth in SEQ ID NO: 43.

In some embodiments, the antigen-binding protein capable of binding to CD73 includes an antibody heavy chain, and the antibody heavy chain includes an amino acid sequence as set forth in SEQ ID NO: 25 or SEQ ID NO: 41.

In some embodiments, the antigen-binding protein capable of binding to CD73 includes at least one CDR in an antibody light chain variable region VL, and the VL includes an amino acid sequence as set forth in SEQ ID NO: 24 or SEQ ID NO: 40.

In some embodiments, the antigen-binding protein capable of binding to CD73 includes LCDR3, and the LCDR3 includes an amino acid sequence as set forth in SEQ ID NO: 17 or SEQ ID NO: 34.

In some embodiments, the antigen-binding protein capable of binding to CD73 includes LCDR2, and the LCDR2 includes an amino acid sequence as set forth in SEQ ID NO: 18 (WAS) or SEQ ID NO: 35 (LAS).

In some embodiments, the antigen-binding protein capable of binding to CD73 includes LCDR1, and the LCDR1 includes an amino acid sequence as set forth in SEQ ID NO: 19 or SEQ ID NO: 36.

In some embodiments, the antigen-binding protein capable of binding to CD73 includes VL, and the VL includes an amino acid sequence as set forth in SEQ ID NO: 24 or SEQ ID NO: 40.

In some embodiments, the antigen-binding protein capable of binding to CD73 includes an antibody light chain constant region.

In some embodiments, the antibody light chain constant region is derived from a human Igκ constant region.

In some embodiments, the antibody light chain constant region includes an amino acid sequence as set forth in SEQ ID NO: 44.

In some embodiments, the antigen-binding protein capable of binding to CD73 includes an antibody light chain, and the antibody light chain includes an amino acid sequence as set forth in SEQ ID NO: 26 or SEQ ID NO: 42.

In some embodiments, the antigen-binding protein capable of binding to CD73 includes VH and VL, and the VH and VL include amino acid sequences selected from the group consisting of:
(1) the VH including an amino acid sequence as set forth in SEQ ID NO: 16, and the VL including an amino acid sequence as set forth in SEQ ID NO: 24; and
(2) the VH including an amino acid sequence as set forth in SEQ ID NO: 33, and the VL including an amino acid sequence as set forth in SEQ ID NO: 40.

In some embodiments, the antigen-binding protein capable of binding to CD73 includes an antibody heavy chain and an antibody light chain, and the antibody heavy chain and the antibody light chain include amino acid sequences selected from the group consisting of:
(1) the antibody heavy chain including an amino acid sequence as set forth in SEQ ID NO: 25, and the antibody light chain including an amino acid sequence as set forth in SEQ ID NO: 26; and
(2) the antibody heavy chain including an amino acid sequence as set forth in SEQ ID NO: 41, and the antibody light chain including an amino acid sequence as set forth in SEQ ID NO: 42.

In another aspect, the present application provides one or more isolated nucleic acid molecules, encoding the isolated antigen-binding protein.

In another aspect, the present application provides a vector, including the nucleic acid molecules.

In another aspect, the present application provides a cell, including the nucleic acid molecule or the vector.

In another aspect, the present application provides a pharmaceutical composition, including the isolated antigen-binding protein, the pharmaceutical combination, the nucleic acid molecule, the vector and/or the cell, and optionally a pharmaceutically acceptable carrier.

In another aspect, the present application provides a method for preparing the isolated antigen-binding protein, including: culturing the cell under a condition that allows expression of the isolated antigen-binding protein.

In another aspect, the present application provides use of the isolated antigen-binding protein, the pharmaceutical combination, the nucleic acid molecule, the vector, the cell and/or the pharmaceutical composition in preparation of a drug, and the drug is used for preventing and/or treating diseases and/or disorders.

In some embodiments, the diseases and/or disorders are mediated by CD39 and/or CD73.

In some embodiments, the diseases and/or disorders include tumors.

In some embodiments, the tumors include solid tumors and/or hematologic tumors.

In some embodiments, the diseases and/or disorders include pancreatic cancer and/or colorectal cancer.

In another aspect, the present application provides a method for detecting CD39 and/or CD73 in a sample, the method includes administration of the isolated antigen-binding protein, the pharmaceutical combination, the nucleic acid molecule, the vector, the cell and/or the pharmaceutical composition.

In some embodiments, the method is an in vitro and/or ex vivo method.

In another aspect, the present application provides use of the isolated antigen-binding protein, the pharmaceutical combination, the nucleic acid molecule, the vector, the cell and/or the pharmaceutical composition in preparation of a kit, and the kit is used for detecting presence of CD39 and/or CD73 in a sample.

Those skilled in the art can easily discern other aspects and advantages of the present application from the following detailed description. Only exemplary embodiments of the present application are shown and described in the following detailed description. As those skilled in the art will realize, the contents of the present application enable those skilled in the art to make changes to the specific embodiments disclosed without departing from the spirit and scope of the invention covered by the present application. Accordingly, the drawings and descriptions in the specification of the present application are illustrative only and not restrictive.

### BRIEF DESCRIPTION OF DRAWINGS

The specific features of the invention to which the present application relates are set forth in the appended claims. The features and advantages of the invention to which the present application relates can be better understood by reference to the exemplary embodiments described in detail below and the drawings. The accompanying drawings are briefly described as follows:
FIG. 1 shows a schematic structural diagram of CD39/CD73 bispecific antibodies 700035 and 700038.
FIG. 2 shows a photograph of a reduced SDS-PAGE gel of the 700035.
FIG. 3 shows a photograph of a reduced SDS-PAGE gel of the 700038.
FIG. 4 shows a photograph of a non-reduced SDS-PAGE gel of the 700035.
FIG. 5 shows a photograph of a non-reduced SDS-PAGE gel of the 700038.
FIG. 6 shows a diagram of curves of enzyme activity of CD39 inhibited by the bispecific antibodies 700035 and 700038.
FIG. 7 shows a diagram of curves of enzyme activity of cell surface CD73 inhibited by the bispecific antibody 700035.
FIG. 8 shows a diagram of curves of enzyme activity of a soluble CD73 antigen inhibited by the bispecific antibody 700038.
FIG. 9 shows the anti-tumor effect of the antigen-binding protein as described in the present application in pancreatic cancer mouse models.
FIG. 10 shows the anti-tumor effect of the antigen-binding protein as described in the present application in colorectal cancer mouse models.

### DETAILED DESCRIPTION

The embodiments of the invention of the present application will be described below with specific examples. Those skilled in the art can easily understand other advantages and effects of the invention of the present application from the disclosure of the specification.

### Definition of Terms

In the present application, the term "CD39" typically refers to an ectonucleoside triphosphate diphosphohydrolase 1 polypeptide encoded by an ENTPD1 gene in a human body. Other names for CD39 include ENTPD1, ATPDase, NTPDase-1 and SPG64. CD39 catalyzes hydrolysis of γ- and β-phosphate residues of extracellular nucleoside triphosphates (NTP, e.g., adenosine triphosphates or ATP) and nucleoside diphosphates (NDP, e.g., adenosine diphosphates or ADP), converting these molecules into nucleoside monophosphate (NMP, e.g., adenosine monophosphate or AMP) derivatives. An exemplary amino acid sequence of CD39 is shown in an NCBI reference sequence: NP_001767.3.

In the present application, the term "CD73" is also called "NT5E", "cluster of differentiation 73", "5'-nucleotidase (5'-NT)", or "5'-nucleotidase ecto". The term covers "full-length", unprocessed CD73, and any form of CD73 produced by cell processing. In the present application, the term "CD73" includes full-length, wild-type CD73 and mutants, fragments, variants, isoforms and homologues thereof. In some embodiments, the CD73 may include human CD73. For example, a sequence for human CD73 can be found under Uniprot login number P21589.

In the present application, the term "isolated" typically refers to artificial collection from a natural state. If an "isolated" substance or component occurs in nature, it may be that the natural environment in which the substance or component is has changed, or that the substance has been isolated from the natural environment, or both. For example, a certain unisolated polynucleotide or polypeptide naturally exists in a living animal, and the same polynucleotide or polypeptide with high purity isolated from the natural state is called "isolated". The term "isolated" does not exclude the presence of artificial or synthetic substances, nor other impure substances that do not affect the activity of the substances.

In the present application, the term "isolated antigen-binding protein" typically refers to a protein which is collected from a natural state by an artificial means and has an antigen-binding ability. The "isolated antigen-binding protein" may include an antigen-binding moiety, and optionally, a framework or frame moiety that allows the antigen-binding moiety to use a conformation that promotes the antigen-binding moiety to bind to an antigen. The antigen-binding protein may comprise, for example, antibody-derived protein framework regions (FRs) or alternative protein framework regions or artificial framework regions with grafted CDRs or CDR derivatives. Such frameworks include, but are not limited to, antibody-derived framework regions including mutations introduced, for example, to stabilize the three-dimensional structure of an antigen-binding protein, as well as fully synthetic framework regions including, for example, biocompatible polymers. Reference may be made, for example, to Korndorfer et al., 2003, Proteins: Structure, Function, and Bioinformatics, 53(1): 121-129 (2003); Roque et al., Biotechnol. Prog. 20:639-654 (2004). Examples of antigen-binding proteins include, but are not limited to: human antibodies and humanized antibodies; chimeric antibodies; recombinant antibodies; single-chain antibodies; bifunctional antibodies; trifunctional antibodies; tetrafunctional antibodies; Fab, Fab', Fv fragments, F(ab')₂, F(ab)₂, scFv, di-scFv and dAb, IgD antibodies; IgE antibodies; IgM antibodies; IgG1 antibodies; IgG2 antibodies; IgG3 antibodies; or IgG4 antibodies and fragments thereof.

In the present application, the isolated antigen-binding protein may include one or more binding domains. In the present application, the binding domain may target different antigens. In the present application, the binding domain may target different epitopes of the same antigen.

In the present application, the terms "variable domain" and "variable region" may be used interchangeably, and typically refer to a moiety of a heavy chain and/or a light chain of an antibody. Variable domains of a heavy chain and a light chain may be referred to as "V_{H}" and "V_{L}" (or "VH" and "VL"), respectively. These domains are typically the most variable moieties of antibodies (relative to other antibodies of the same type), and include antigen-binding sites.

In the present application, the term "variable" typically refers to a fact that there is a considerable difference in sequences of certain segments of variable domains between antibodies. A variable domain mediates antigen binding and determines the specificity of a specific antibody for its specific antigen. However, variability is not evenly distributed across an entire variable domain. The variability is typically concentrated in three segments called hypervariable regions (CDRs or HVRs) in light and heavy chain variable domains. A more highly conserved moiety of a variable domain is called a framework region (FR). The variable domains of the natural heavy and light chains each contain four FR regions, most of which are in a β-fold configuration, connected by three CDRs, which form a ring connection and, in some cases, a part of the β-fold structure. The CDRs in each chain are held together closely by FR regions, and the CDRs from another chain together promote formation of an antigen-binding site on an antibody (see Kabat et al, Sequences of Immunological Interest, Fifth Edition, National Institute of Health, Bethesda, Md. (1991)).

In the present application, the term "antibody" typically refers to an immunoglobulin or a fragment or derivative thereof, and covers any polypeptide that includes an antigen-binding site, whether produced in vitro or in vivo. The term "antibody" includes, but is not limited to, a polyclonal, monoclonal, monospecific, multispecific, non-specific, humanized, single chain, chimeric, synthetic, recombinant, hybridized, mutated, and transplanted antibody. Unless otherwise modified by the term "complete", e.g., in a "complete antibody", for purposes of the present invention, the term "antibody" also includes an antibody fragment, such as Fab, F(ab')₂, Fv, scFv, Fd, dAb and any other antibody fragment that maintains an antigen-binding function (e.g., specifically binding to CLDN18.2). Typically, such a fragment should include an antigen-binding domain. The basic 4-chain antibody unit is a heterotetrameric glycoprotein consisting of two identical light (L) chains and two identical heavy (H) chains. IgM antibodies consist of 5 basic heterotetrameric units and another polypeptide called a J chain and has 10 antigen-binding sites, while IgA antibodies consist of 2-5 basic 4-chain units that can be combined with the J chain to polymerize to form a polyvalent combination. In a case of IgG, the 4-chain unit is generally about 150,000 daltons. Each L chain is connected to the H chain by one covalent disulfide bond, while the two H chains are connected to each other by one or more disulfide bonds depending on the H chain isoform. Each H and L chain also has regularly spaced intra-chain disulfide bridging bonds. Each H strand has a variable domain (VH) at an N-terminus, followed by three constant domains (CH) for α and γ chains, and four CH domains for µ and ε isoforms. Each L chain has a variable domain (VL) at the N-terminus and a constant domain at its other end. The VL corresponds to the VH, and CL corresponds to a first constant domain (CH1) of the heavy chain. Specific amino acid residues are treated to form an interface between the light and heavy chain variable domains. The VH and VL pair together to form a single antigen binding site. The structure and properties of different classes of antibodies refer to, for example, page 71 and Chapter 6 in Basic and Clinical Immunology, 8th Edition, Daniel P. Sties, Abba I. Terr and Tristram G. Parsolw (eds), Appleton & Lange, Norwalk, Conn., 1994. The L chain from any vertebrate species may be classified into one of two distinct types, called κ and λ, based on the amino acid sequence of a constant domain of the L chain. According to the amino acid sequence of a constant domain of a heavy chain (CH), immunoglobulins may be classified into different categories or isotypes. There are currently five categories of immunoglobulins: IgA, IgD, IgE, IgG, and IgM, with the heavy chains named α, δ, ε, γ, and µ, respectively. Based on relatively small differences in CH sequence and function, the γ and α types are further divided into subtypes. For example, humans express the following subtypes: IgG1, IgG2A, IgG2B, IgG3, IgG4, IgA1, and IgK1.

In the present application, the term "CDR", also known as a "complementarity determining region", typically refers to a region in an antibody variable domain, the sequence of which is highly variable and/or forms a structure defining loop. For example, an antibody may include 6 CDRs, 3 in VH (HCDR1, HCDR2, HCDR3), and 3 in VL (LCDR1, LCDR2, LCDR3). In some embodiments, a naturally occurring camel antibody consisting only of heavy chains can function normally and stably even in the absence of light chains. Reference may be made, for example, to Hamers-Casterman et al., Nature 363:446-448 (1993); Sheriff et al, Nature Struct. Biol. 3:733-736 (1996).

In the present application, the term "FR" typically refers to a more highly conserved moiety of an antibody variable domain, called a framework region. Typically, a variable domain of a natural heavy and/or light chain each includes 4 FR regions, i.e., 4 in VH (H-FR1, H-FR2, H-FR3, and H-FR4), and/or 4 in VL (L-FR1, L-FR2, L-FR3, and L-FR4).

In the present application, the term "antigen-binding fragment" typically refers to one or more fragments capable of specifically binding to antigens. In the present application, the antigen-binding fragment may include Fab, Fab', F(ab)₂, Fv fragments, F(ab')₂, scFv, di-scFv and/or dAb.

In the present application, the term "Fab" generally refers to antigen-binding fragments of antibodies. As described above, intact antibodies can be digested using papain. After digestion with papain, an antibody produces two identical antigen-binding fragments, namely a "Fab" fragment and a residual "Fc" fragment (i.e. Fc region, as above). A Fab fragment may consist of one complete L chain, variable region of a heavy chain, and the first constant region (C_{H}1) of the H chain (V_{H}).

In the present application, the term "Fab' fragment" typically refers to a monovalent antigen-binding fragment of a human monoclonal antibody, which is slightly larger than an Fab fragment. For example, an Fab' fragment may include all light chains, all heavy chain variable regions, and all or part of the first and second constant regions of the heavy chains. For example, an Fab' fragment may also include part or all of 220-330 amino acid residues in the heavy chains.

In the present application, the term "F(ab')2" typically refers to an antibody fragment produced by digesting an intact antibody with gastric protease. The F(ab')2 fragment contains two Fab fragments held together by disulfide bonds and part of the hinge region. F(ab')2 fragments have bivalent antigen-binding activity and are capable of cross-linking antigens.

In the present application, the term "Fv fragment" generally refers to a monovalent antigen-binding fragment of a human monoclonal antibody, including all or part of heavy chain variable regions and light chain variable regions, and lacking heavy chain constant regions and light chain constant regions. A heavy chain variable region and a light chain variable region include, for example, CDRs. For example, an Fv fragment includes all or part of amino terminal variable regions of about 110 amino acids in both heavy and light chains.

In the present application, the term "scFv" typically refers to a fusion protein including at least one antibody fragment including a light chain variable region and at least one antibody fragment including a heavy chain variable region, wherein the light chain and heavy chain variable regions are adjacent (e.g. via a synthetic linker such as a short flexible polypeptide linker) and can be expressed in the form of a single chain polypeptide, and the scFv retains the specificity of the intact antibody where it is derived. Unless otherwise specified, as used in the present application, scFv may include VL and VH variable regions in any order (e.g. relative to the N-terminus and C-terminus of a polypeptide), and scFv may include VL-linker-VH or VH-linker-VL.

In the present application, the term "dAb" typically refers to an antigen-binding fragment having a VH domain and a VL domain, or having a VH domain or a VL domain, referring to, for example, Ward et al. (Nature, 1989 Oct 12; 341 (6242): 544-6); referring to Holt et al., Trends Biotechnol., 2003, 21(11): 484-490; and referring to other published patent applications such as WO 06/030220, WO 06/003388, and DomantisLtd.

In the present application, the term "monoclonal antibody" typically refers to an antibody molecule preparation consisting of single molecules. Monoclonal antibodies are usually highly specific for a single antigenic site. Furthermore, unlike conventional polyclonal antibody preparations, which often have different antibodies directed against different determinants, each monoclonal antibody is directed against a single determinant on the antigen. In addition to their specificity, the advantage of monoclonal antibodies is that they can be synthesized by hybridoma cultures without contamination by other immunoglobulins. The modifier "monoclonal" represents the characteristics of antibodies obtained from a substantially homogeneous antibody population and is not interpreted as requiring the production of antibodies by any particular method. For example, the monoclonal antibodies used herein can be produced in hybridoma cells or can be produced by recombinant DNA methods.

In the present application, the term "chimeric antibody" typically refers to an antibody in which a variable region is derived from one species and a constant region is derived from another. Typically, the variable region is derived from antibodies of experimental animals such as rodents ("parental antibodies"), and the constant region is derived from human antibodies, such that the resulting chimeric antibody is less likely to elicit an adverse immune response in a human individual than the parental (e.g., mouse-derived) antibodies.

In the present application, the term "humanized antibody" typically refers to an antibody in which part or all of the amino acids outside the CDR region of a non-human antibody (e.g., a mouse antibody) are substituted with corresponding amino acids derived from human immunoglobulins. In the CDR region, small additions, deletions, insertions, substitutions, or modifications of amino acids are also allowed as long as they retain the ability of the antibody to bind to specific antigens. The humanized antibody may optionally comprise at least a part of the human immunoglobulin constant region. The "humanized antibody" retains antigen specificity similar to the original antibody. The "humanized" form of a non-human (e.g., mouse) antibody may contain a chimeric antibody derived from a sequence of a non-human immunoglobulin to a minimum extent. In some cases, CDR region residues in a human immunoglobulin (receptor antibody) may be substituted with CDR region residues of a non-human species (donor antibody) (e.g., mouse, rat, rabbit or non-human primate) having the desired property, affinity and/or ability. In some cases, FR region residues of a human immunoglobulin may be substituted with corresponding non-human residues. Furthermore, humanized antibodies may comprise amino acid modifications that are not found in the receptor antibody or in the donor antibody. These modifications may be made to further improve the properties of the antibody, e.g., binding affinity.

The term "fully human antibody" typically refers to an antibody that includes only a protein sequence of a human immunoglobulin. If produced in mice, in mouse cells, or in hybridomas derived from mouse cells, a fully human antibody may include a mouse sugar chain. Similarly, a "mouse antibody" or a "rat antibody" refers to an antibody that includes only a sequence of a mouse or rat immunoglobulin, respectively. A fully human antibody may be produced in a human body, or in a transgenic animal having a germline sequence of a human immunoglobulin, by phage display or other molecular biological methods. Exemplary techniques for producing the antibody are described in US patents 6150584, 6458592, and 6420140. Other techniques, such as use of libraries, are known in the art.

In the present application, the term "directly linked" is opposite to the term "indirectly linked", and the term "directly linked" typically refers to a direct linking. For example, the direct connection may be situations that substances are directly connected without spacers. The spacer may be a linker. For example, the linker may be a peptide linker. The term "indirect connection" usually refers to situations that substances are not directly connected to each other. For example, the indirect linking may be a case of linking via a spacer. For example, in the isolated antigen-binding protein as described in the present application, the C-terminus of L-FR1 may be directly or indirectly linked to the N-terminus of LCDR1.

In the present application, the term "pharmaceutical combination" typically refers to a combination that includes at least two active ingredients/therapeutic agents. In some embodiments, each active ingredients/therapeutic agents may be prepared as separate preparations (solid, liquid, gel, etc.). In some embodiments, each active ingredients/therapeutic agents may be contained in different containers, and may also be prepared simultaneously or separately with suitable carriers into desired preparations when necessary. In some embodiments, each active ingredients/therapeutic agents may be of different sources. In some embodiments, each active ingredients/therapeutic agents may be prepared into a pharmaceutical composition in a mixture form.

In the present application, the term "isolated nucleic acid molecule" typically refers to a nucleotide, deoxyribonucleotide or ribonucleotide in an isolated form and of any length, or an analogue isolated from its natural environment or artificially synthesized.

In the present application, the term "vector" generally refers to a nucleic acid carrying tool into which polynucleotide encoding a certain protein can be inserted and the protein is expressed. A vector may be transformed, transduced, or transfected into a host cell to express a genetic material element it carries within the host cell. For example, vectors may include plasmids; phagemids; Cosmids; artificial chromosomes such as yeast artificial chromosomes (YAC), bacterial artificial chromosomes (BAC), or P1 derived artificial chromosomes (PAC); phages such as λ phage or M13 phage; animal viruses; and the like. The types of animal viruses used as vectors include retroviruses (including lentiviruses), adenoviruses, adeno-associated viruses, herpes viruses (e.g., herpes simplex virus), poxviruses, baculoviruses, papillomaviruses, and papovaviruses (e.g., SV40). A vector may include a variety of elements that control expression, including a promoter sequence, a transcription initiation sequence, an enhancer sequence, a selection element, and a reporter gene. In addition, the vector may also contain a replication initiation site. The vector may also include components that facilitate to enter cells, such as viral particles, liposomes, or protein coats, but not just these.

In the present application, the term "cell" generally refers to a single cell, cell line, or cell culture that may be, or has been a recipient of a subject plasmid or vector, which includes thenucleic acid molecule according to the present application or the vector according to the present application. The cell can include the progeny of a single cell. Due to natural, accidental, or intentional mutations, the progeny may not necessarily be exactly the same as the original parental cell (in the morphology of the total DNA complement or in the genome). The cell may include a cell transfected in vitro using the vector described in the present application. The cell may be a bacterial cell (e.g., E. coli), a yeast cell, or other eukaryotic cells, e.g., a COS cell, a Chinese hamster ovary (CHO) cell, a CHO-K1 cell, a LNCAP cell, a HeLa cell, a HEK293 cell, a COS-1 cell, or a NS0 cell. In some embodiments, the cell is a mammalian cell.

In the present application, the term "pharmaceutical composition" typically refers to a composition for use in prevention/treatment of diseases or disorders. The pharmaceutical composition may include the isolated antigen-binding protein as described in the present application, the nucleic acid molecule as described the present application, the vector as described the present application, and/or the cell as described the present application, and optionally a pharmaceutically acceptable adjuvant. In addition, the pharmaceutical composition may include one or more suitable preparations of a (pharmaceutically effective) carrier, stabilizer, excipient, diluent, solubilizer, surfactant, emulsifier and/or preservative. The acceptable components of the composition are preferably non-toxic to the recipient at the doses and concentrations used. The pharmaceutical composition of the present application includes, but is not limited to, a liquid, frozen or freeze-dried composition.

In the present application, the term "pharmaceutically acceptable carrier" typically includes a pharmaceutically acceptable carrier, excipient or stabilizer that is non-toxic at the dose and concentration used to a cell or mammal exposed thereto. A physiologically acceptable carrier may include, for example, a buffering agent; antioxidant; low molecular weight (less than about 10 residues) polypeptide; protein; hydrophilic polymer; amino acid; monosaccharide, disaccharide and any other carbohydrate; chelating agent; sugar alcohol; salt forming counter ion, e.g., sodium; and/or non-ionic surfactant.

In the present application, the term "specific binding" or "specific" typically refers to measurable and reproducible interactions, e.g., the binding between a target and an antibody, which can determine the presence of a target in the presence of a heterogeneous population of molecules (including biomolecules). For example, an antibody that specifically binds to a target (which may be an epitope) may be an antibody that binds to the target with greater affinity, avidity, ease, and/or longer duration than those when binding to other targets. In some embodiments, an antibody specifically binds to an epitope on a protein, and the epitope is conserved across different species of proteins. In some embodiments, specific binding may include, but does not require exclusive binding.

In the present application, the term "subject" typically refers to a human or a non-human animal, including, but is not limited to a cat, dog, horse, pig, cow, sheep, rabbit, mouse, rat, or monkey.

In the present application, the term "tumor" typically refers to a neoplasm or solid lesion formed by abnormal cell growth. In the present application, a tumor may be a solid tumor or non-solid tumor.

In the present application, a protein, polypeptide, and/or amino acid sequence involved should also be understood to include at least the following scope: variants or homologues having the same or similar functions as the protein or polypeptide.

In the present application, the variants may be, for example, proteins or polypeptides with substitution, deletion or addition of one or more amino acids in the amino acid sequence of the protein and/or polypeptide (e.g., an antibody or fragment thereof specifically binding to a CD73 and/or CD39 protein). For example, the functional variants may include proteins or polypeptides with amino acid alterations through substitution, deletion and/or insertion of at least 1, e.g., 1-30, 1-20, 1-10, 1, 2, 3, 4 or 5 amino acids. The functional variant may substantially retain the biological properties of the protein or polypeptide prior to the alteration (e.g., substitution, deletion, or addition). For example, the functional variant may retain at least 60%, 70%, 80%, 90%, or 100% of the biological activity (e.g., antigen-binding ability) of the protein or polypeptide prior to the alteration. For example, the substitution may be conservative substitution.

In the present application, the homologues may be proteins or polypeptides having at least about 85% (e.g., having at least about 85%, about 90%, about 91%, about 92%, about 93%, about 94%, about 95%, about 96%, about 97%, about 98%, about 99% or higher) sequence homology with the amino acid sequence of the protein and/or polypeptide (e.g., an antibody or fragment thereof specifically binding to a CD73 and/or CD39 protein).

In the present application, the homology typically refers to similarity, likeness or association between two or more sequences. "Percent sequence homology" can be calculated by comparing two sequences to be aligned in a comparison window to determine the number of positions with identical nucleic acid bases (e.g., A, T, C, G, I) or identical amino acid residues (e.g., Ala, Pro, Ser, Thr, Gly, Val, Leu, Ile, Phe, Tyr, Trp, Lys, Arg, His, Asp, Glu, Asn, Gln, Cys, and Met) in the two sequences to obtain the number of matching positions, dividing the number of matching positions by the total number of positions in the comparison window (i.e., window size), and multiplying the result by 100 to generate the percent sequence homology. Alignment for the purpose of determining percent sequence homology can be accomplished in a variety of ways known in the art, for example, using publicly available computer software such as BLAST, BLAST-2, ALIGN or Megalign (DNASTAR) software. One skilled in the art can determine appropriate parameters for aligning sequences, including any algorithms required to achieve maximal alignment within the full-length sequences being compared or within the sequence region of interest. The homology can also be determined by the following methods: FASTA and BLAST. A description of the FASTA algorithm can be found in W. R. Pearson and D. J. Lipman, "Improved Tool for Biological Sequence Comparison", (Proc. Natl. Acad. Sci. ), 85: 2444-2448, 1988; and D. J. Lipman and W. R. Pearson, "Fast and Sensitive Protein Similarity Search", Science, 227: 1435-1441, 1989. A description of the BLAST algorithm can be found in S. Altschul, W. Gish, W. Miller, E. W. Myers and D. Lipman, "Basic Local Alignment Search Tool", Journal of Molecular Biology, 215: 403-410, 1990.

In the present application, the term "include" typically refers to the meaning of include, comprise, contain, or encompass. In some cases, it also means "be" and "consist of".

In the present application, the term "about" typically refers to a change in a range of 0.5-10% greater or less than a specified value, e.g., a change in a range of 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, or 10% greater or less than the specified value.

### DETAILED DESCRIPTION OF THE INVENTION

In one aspect, the present application provides an isolated antigen-binding protein, which includes a first binding domain and a second binding domain, wherein the first binding domain is capable of binding to a CD39 protein, and the second binding domain is capable of binding to a CD73 protein.

In the present application, the isolated antigen-binding protein may have one or more of the following properties: (1) capability of binding to a CD39 protein at a K_{D} value of 1×10⁻⁹ M or lower; (2) capability of binding to a CD73 protein at a K_{D} value of 1×10⁻⁹ M or lower; (3) inhibitory effect on enzymatic activity of a CD39 antigen; (4) inhibitory effect on enzymatic activity of a CD73 antigen; (5) thermal stability; (6) capability of binding to human CD39 and/or cynomolgus CD73; (7) capability of binding to human CD73 and/or cynomolgus CD73; and (8) capability of inhibiting growth of tumor cells.

For example, the tumor may include a CD39 positive tumor. For example, the tumor may include a CD73 positive tumor. For example, the tumor may include a solid tumor. For example, the tumor may include a non-solid tumor.

In the present application, the isolated antigen-binding protein is capable of binding to a CD39 protein at a K_{D} value of 1×10⁻⁹ M or lower. For example, the isolated antigen-binding protein is capable of binding to a CD39 protein at a K_{D} value of less than about 1×10⁻⁹ M, less than about 8×10⁻¹⁰ M, less than about 6× 10⁻¹⁰ M, less than about 4×10⁻¹⁰ M, less than about 2×10⁻¹⁰ M, less than about 1×10⁻¹⁰ M or lower.

In the present application, the isolated antigen-binding protein is capable of inhibiting ATPase activity in CD39 expressing cells at an EC50 less than about 0.8 µg/mL according to an ATPase activity analysis.

For example, the EC50 may be less than about 0.7 µg/mL, less than about 0.6 µg/mL, less than about 0.5 µg/mL, less than about 0.4 µg/mL, less than about 0.3 µg/mL, less than about 0.2 µg/mL, less than about 0.1 µg/mL, less than about 0.09 µg/mL, less than about 0.08 µg/mL, less than about 0.07 µg/mL, less than about 0.06 µg/mL, less than about 0.05 µg/mL, less than about 0.04 µg/mL, less than about 0.03 µg/mL, less than about 0.02 µg/mL, less than about 0.01 µg/mL, or lower.

A CDR of an antibody, also known as a complementarity determining region, is part of a variable region. An amino acid residue in this region may come into contact with an antigen or antigenic epitope. An antibody CDR may be determined by a variety of encoding systems, e.g., CCG, Kabat, Chothia, IMGT, and Kabat/Chothia considered comprehensively. These encoding systems are known in the art, and reference may be made to http://www.bioinf.org.uk/abs/index.html#kabatnum for details. Those skilled in the art may use different encoding systems to determine a CDR region based on the sequence and structure of an antibody. Using different encoding systems, the CDR regions may differ. In the present application, the CDR encompasses CDR sequences divided according to any CDR division method; the CDR also encompasses variants thereof including amino acid sequences of the CDR after substitution, deletion and/or addition of one or more amino acids, e.g., substitution, deletion and/or insertion of 1-30, 1-20, 1-10, 1, 2, 3, 4, 5, 6, 7, 8 or 9 amino acids. The CDR also encompasses the homologues thereof which may have the amino acid sequences having at least about 85% (e.g., having at least about 85%, about 90%, about 91%, about 92%, about 93%, about 94%, about 95%, about 96%, about 97%, about 98%, about 99% or higher) sequence homology with the amino acid sequence of the CDR.

In the present application, for example, the CDR may be divided in an IMGT manner.

In addition, it is to be noted that the isolated antigen-binding protein as described in the present application may include a heavy chain and/or light chain sequence having one or more conserved sequence modifications present in the antigen-binding protein. The "conserved sequence modifications" refer to amino acid modifications that do not significantly affect or change the antibody binding properties. Such conserved modifications include amino acid substitutions, additions, and deletions. The modification can be introduced into the isolated antigen-binding proteins according to the present application by standard techniques known in the art, such as point mutations and PCR-mediated mutations. The conserved amino acid substitution refers to the substitution of amino acid residues with amino acid residues having similar side chains. The group of amino acid residues having similar side chains is known in the art. Such groups of amino acid residues include amino acids with basic side chains (e.g., lysine, arginine and histidine), acidic side chains (e.g., aspartic acid and glutamic acid), side chains without polarity (e.g., glycine, asparagine, glutamine, serine, threonine, tyrosine, cysteine and tryptophan), nonpolar side chains (e.g., alanine, valine, leucine, isoleucine, proline, phenylalanine and methionine), β-branched side chains (e.g., threonine, valine and isoleucine), and aromatic side chains (e.g., tyrosine, phenylalanine, tryptophan and histidine). In the present application, one or more amino acid residues in the CDR region of the isolated antigen-binding protein as described in the present application may be substituted with other amino acid residues in the same side chain group. Those skilled in the art know that some conserved sequence modifications do not cause loss of antigen binding activity, and for details, reference may be made, for example, to Brummell et al., (1993) Biochem 32:1180-8; de Wildt et al., (1997) Prot. Eng. 10:835-41; Komissarov et al., (1997) J. Biol. Chem. 272:26864-26870; Hall et al., (1992) J. Immunol. 149:1605-12; Kelley and O'Connell (1993) Biochem.32:6862-35; Adib-Conquy et al., (1998) Int. Immunol.10:341-6 and Beers et al., (2000) Clin. Can. Res. 6:2835-43.

The CD39 antigen-binding protein as described in the present application may be identified, screened or characterized by all kinds of assays known in the art.

For example, the antigen-binding activity of the antigen-binding protein or fusion protein of the present application may be tested by known methods, such as enzyme-linked immunosorbent assay (ELISA), immunoblotting (e.g., protein blotting), flow cytometry (e.g., FACS), immunohistochemistry, and immunofluorescence.

In the present application, the first binding domain of the isolated antigen-binding protein may be directly or indirectly linked to the second binding domain.

In the present application, the C-terminus of the first binding domain of the isolated antigen-binding protein may be linked to the N-terminus of the second binding domain.

In the present application, the C-terminus of the first binding domain of the isolated antigen-binding protein may be linked to the N-terminus of a heavy chain variable region of the second binding domain.

In the present application, the first binding domain of the isolated antigen-binding protein may be linked to the second binding domain by a linker. In the present application, the linker may include a peptide linker. In the present application, the linker may include an amino acid sequence of (GGGGS) n, where n is any positive integer from 0 to 10. For example, the linker may include an amino acid sequence as set forth in SEQ ID NO: 47.

In the present application, the isolated antigen-binding protein may include two of the first binding domains.

In the present application, the two first binding domains of the isolated antigen-binding protein may be directly or indirectly linked to the N-terminus of the second binding domain respectively.

In the present application, the two first binding domains of the isolated antigen-binding protein may be directly or indirectly linked to the N-termini of two heavy chains of the second binding domain respectively.

In the present application, the isolated antigen-binding protein may include a first polypeptide chain and a second polypeptide chain, wherein the first polypeptide chain may include heavy chains of the first binding domain and the second binding domain.

In the present application, the N-termini of the heavy chains of the first binding domain and the second binding domain of the isolated antigen-binding protein may be directly or indirectly connected.

In the present application, the N-termini of the heavy chains of the first binding domain and the second binding domain of the isolated antigen-binding protein may be connected by a linker.

In the present application, the first polypeptide chain of the isolated antigen-binding protein may include an amino acid sequence as set forth in SEQ ID NO: 45 or SEQ ID NO: 46.

In the present application, the second polypeptide chain of the isolated antigen-binding protein may include a light chain of the second binding domain.

In the present application, the second polypeptide chain of the isolated antigen-binding protein may include an amino acid sequence as set forth in SEQ ID NO: 26 or SEQ ID NO: 42.

In the present application, the isolated antigen-binding protein may include two of the first polypeptide chains and two of the second polypeptide chains.

### Pharmaceutical combination

In the present application, a pharmaceutical combination is further provided, including an antigen-binding protein capable of binding to CD39 and an antigen-binding protein capable of binding to CD73.

In the present application, the antigen-binding protein capable of binding to CD39 and the antigen-binding protein capable of binding to CD73 in the pharmaceutical combination may be co-administered or separately administered; may be administered in the same manner or in a different manner; and may be administered simultaneously or sequentially (the order of administration may not be limited).

In the present application, the antigen-binding protein capable of binding to CD39 and the antigen-binding protein capable of binding to CD73 in the pharmaceutical combination may be contained in the same container or in different containers; and may be independently packaged or be mixed.

In the present application, the antigen-binding protein capable of binding to CD39 has one or more of the following properties:
(1) capability of binding to human CD39 at an EC50 less than about 0.5 µg/mL according to an FACS detection method, for example, the EC50 may be less than about 0.4 µg/mL, less than about 0.3 µg/mL, less than about 0.2 µg/mL, less than about 0.1 µg/mL, less than about 0.09 µg/mL, less than about 0.08 µg/mL, less than about 0.07 µg/mL, less than about 0.06 µg/mL, less than about 0.05 µg/mL, less than about 0.04 µg/mL, less than about 0.03 µg/mL, less than about 0.02 µg/mL, less than about 0.01 µg/mL, or lower;
(2) capability of binding to cynomolgus (cyno) CD39 at an EC50 less than about 0.7 µg/mL according to an FACS detection method, for example, the EC50 may be less than about 0.6 µg/mL, less than about 0.5 µg/mL, less than about 0.4 µg/mL, less than about 0.3 µg/mL, less than about 0.2 µg/mL, less than about 0.1 µg/mL, less than about 0.09 µg/mL, less than about 0.08 µg/mL, less than about 0.07 µg/mL, less than about 0.06 µg/mL, less than about 0.05 µg/mL, less than about 0.04 µg/mL, less than about 0.03 µg/mL, less than about 0.02 µg/mL, less than about 0.01 µg/mL, or lower;
(3) capability of binding to human CD39 at a KD less than about 1.3×10⁻⁸ M according to a Biacore detection method, for example, the KD may be less than about 1×10⁻⁸ M, less than about 9×10⁻⁹ M, less than about 8×10⁻⁹ M, less than about 7×10⁻⁹ M, less than about 6×10⁻⁹ M, less than about 5×10⁻⁹ M, less than about 4×10⁻⁹ M, less than about 3×10⁻⁹ M, less than about 2×10⁻⁹ M, less than about 1×10⁻⁹ M, less than about 8×10⁻¹⁰ M, less than about 6×10⁻¹⁰ M, less than about 4×10⁻¹⁰ M, less than about 2×10⁻¹⁰ M, less than about 1×10⁻¹⁰ M, or lower;
(4) capability of binding to cynomolgus (cyno) CD39 at a KD less than about 1.1×10⁻⁷ M according to a Biacore detection method, for example, the KD may be less than about 1×10⁻⁷ M, less than about 9×10⁻⁸ M, less than about 8×10⁻⁸ M, less than about 7×10⁻⁸ M, less than about 6×10⁻⁸ M, less than about 5×10⁻⁸ M, less than about 4×10⁻⁸ M, less than about 3×10⁻⁸ M, less than about 2×10⁻⁸ M, less than about 1×10⁻⁸ M, less than about 8×10⁻⁹ M, less than about 6×10⁻⁹ M, less than about 4×10⁻⁹ M, less than about 2×10⁻⁹ M, less than about 1×10⁻⁹ M, or lower;
(5) capability of inhibiting ATPase activity in CD39 expressing cells at an EC50 less than about 0.8 µg/mL according to an ATPase activity analysis, for example, the EC50 may be less than about 0.7 µg/mL, less than about 0.6 µg/mL, less than about 0.5 µg/mL, less than about 0.4 µg/mL, less than about 0.3 µg/mL, less than about 0.2 µg/mL, less than about 0.1 µg/mL, less than about 0.09 µg/mL, less than about 0.08 µg/mL, less than about 0.07 µg/mL, less than about 0.06 µg/mL, less than about 0.05 µg/mL, less than about 0.04 µg/mL, less than about 0.03 µg/mL, less than about 0.02 µg/mL, less than about 0.01 µg/mL, or lower; and
(6) capability of inhibiting the function of CD39 of tumor cells.

In the present application, the antigen-binding protein capable of binding to CD73 has one or more of the following properties:
(1) capability of specifically binding to CD73 or a functional active fragment thereof;
(2) capability of inhibiting the enzymatic activity of CD73;
(3) capability of inducing endocytosis of CD73 on the cell surface; and
(4) capability of inhibiting growth and/or proliferation of tumor cells.

### First binding domain

In the present application, the first binding domain is capable of binding to a CD39 protein. For example, the first binding domain may include an antigen-binding protein capable of binding to CD39.

In the present application, the first binding domain may include an antibody or an antigen-binding fragment thereof.

In the present application, the antibody may be selected from the group consisting of a monoclonal antibody, a single-chain antibody, a chimeric antibody, a multispecific antibody, a humanized antibody, and a fully human antibody. In the present application, the antigen-binding fragment may be selected from the group consisting of: Fab, Fab', F(ab)₂, Fv, F(ab')₂, scFv, VHH, di-scFv and dAb fragments.

In the present application, the first binding domain of the isolated antigen-binding protein may be VHH.

In the present application, the first binding domain of the isolated antigen-binding protein includes at least one CDR in an antibody heavy chain variable region VHH, and the VHH includes an amino acid sequence as set forth in SEQ ID NO: 8.

In the present application, the first binding domain may include CDR3, and the CDR3 may include an amino acid sequence as set forth in SEQ ID NO: 1.

In the present application, the first binding domain may include CDR2, and the CDR2 may include an amino acid sequence as set forth in SEQ ID NO: 2.

In the present application, the first binding domain may include CDR1, and the CDR1 may include an amino acid sequence as set forth in SEQ ID NO: 3.

In the present application, the first binding domain may include CDR1, CDR2 and CDR3, the CDR1 may include an amino acid sequence as set forth in SEQ ID NO: 3, the CDR2 may include an amino acid sequence as set forth in SEQ ID NO: 2, and the CDR3 may include an amino acid sequence as set forth in SEQ ID NO: 1.

In the present application, the first binding domain may include FR1, the C-terminus of the FR1 is directly or indirectly linked to the N-terminus of the CDR1, and the FR1 may include an amino acid sequence as set forth in SEQ ID NO: 4.

In the present application, the first binding domain may include FR2, the FR2 is between the CDR1 and the CDR2, and the FR2 may include an amino acid sequence as set forth in SEQ ID NO: 5.

In the present application, the first binding domain may include FR3, the FR3 is between the CDR2 and the CDR3, and the FR3 may include an amino acid sequence as set forth in SEQ ID NO: 6.

In the present application, the first binding domain may include FR4, the N-terminus of the FR4 is directly or indirectly linked to the C-terminus of the CDR3, and the FR4 may include an amino acid sequence as set forth in SEQ ID NO: 7.

In the present application, the first binding domain may include VHH, and the VHH may include an amino acid sequence as set forth in SEQ ID NO: 8.

### Second binding domain

In the present application, the second binding domain is capable of binding to a CD73 protein. In the present application, the second binding domain may include an antigen-binding protein capable of binding to CD73.

In the present application, the second binding domain may include an antibody or an antigen-binding fragment thereof.

In the present application, the antibody may be selected from the group consisting of a monoclonal antibody, a single-chain antibody, a chimeric antibody, a multispecific antibody, a humanized antibody, and a fully human antibody.

In the present application, the antigen-binding fragment may be selected from the group consisting of: Fab, Fab', F(ab)₂, Fv, F(ab')₂, scFv, VHH, di-scFv and dAb fragments.

In the present application, the second binding domain may include at least one CDR in an antibody heavy chain variable region VH, and the VH includes an amino acid sequence as set forth in SEQ ID NO: 16 or SEQ ID NO: 33.

In the present application, the second binding domain may include HCDR3, and the HCDR3 may include an amino acid sequence as set forth in SEQ ID NO: 9 or SEQ ID NO: 27.

In the present application, the second binding domain may include HCDR2, and the HCDR2 may include an amino acid sequence as set forth in SEQ ID NO: 10 or SEQ ID NO: 28.

In the present application, the second binding domain may include HCDR1, and the HCDR1 may include an amino acid sequence as set forth in SEQ ID NO: 11 or SEQ ID NO: 29.

In the present application, the second binding domain may include HCDR1, HCDR2 and HCDR3, the HCDR1 may include an amino acid sequence as set forth in SEQ ID NO: 11 or SEQ ID NO: 29, the HCDR2 may include an amino acid sequence as set forth in SEQ ID NO: 10 or SEQ ID NO: 28, and the HCDR3 may include an amino acid sequence as set forth in SEQ ID NO: 9 or SEQ ID NO: 27. For example, the HCDR1 may include an amino acid sequence as set forth in SEQ ID NO: 11, the HCDR2 may include an amino acid sequence as set forth in SEQ ID NO: 10, and the HCDR3 may include an amino acid sequence as set forth in SEQ ID NO: 9. For example, the HCDR1 may include an amino acid sequence as set forth in SEQ ID NO: 29, the HCDR2 may include an amino acid sequence as set forth in SEQ ID NO: 28, and the HCDR3 may include an amino acid sequence as set forth in SEQ ID NO: 27.

In the present application, the second binding domain may include H-FR1, the C-terminus of the H-FR1 is directly or indirectly linked to the N-terminus of the HCDR1, and the H-FR1 may include an amino acid sequence as set forth in SEQ ID NO: 12 or SEQ ID NO: 30.

In the present application, the second binding domain may include H-FR2, the H-FR2 is between the HCDR1 and the HCDR2, and the H-FR2 may include an amino acid sequence as set forth in SEQ ID NO: 13 or SEQ ID NO: 31.

In the present application, the second binding domain may include H-FR3, the H-FR3 is between the HCDR2 and the HCDR3, and the H-FR3 may include an amino acid sequence as set forth in SEQ ID NO: 14 or SEQ ID NO: 32.

In the present application, the second binding domain may include H-FR4, the N-terminus of the H-FR4 is directly or indirectly linked to the C-terminus of the HCDR3, and the H-FR4 may include an amino acid sequence as set forth in SEQ ID NO: 15.

In the present application, the second binding domain may include H-FR1, H-FR2, H-FR3 and H-FR4, the H-FR1 may include an amino acid sequence as set forth in SEQ ID NO: 12 or SEQ ID NO: 30, the H-FR2 may include an amino acid sequence as set forth in SEQ ID NO: 13 or SEQ ID NO: 31, the H-FR3 may include an amino acid sequence as set forth in SEQ ID NO: 14 or SEQ ID NO: 32, and the H-FR4 may include an amino acid sequence as set forth in SEQ ID NO: 15. For example, the H-FR1 may include an amino acid sequence as set forth in SEQ ID NO: 12, the H-FR2 may include an amino acid sequence as set forth in SEQ ID NO: 13, the H-FR3 may include an amino acid sequence as set forth in SEQ ID NO: 14, and the H-FR4 may include an amino acid sequence as set forth in SEQ ID NO: 15. For example, the H-FR1 may include an amino acid sequence as set forth in SEQ ID NO: 30, the H-FR2 may include an amino acid sequence as set forth in SEQ ID NO: 31, the H-FR3 may include an amino acid sequence as set forth in SEQ ID NO: 32, and the H-FR4 may include an amino acid sequence as set forth in SEQ ID NO: 15.

In the present application, the second binding domain may include VH, and the VH may include an amino acid sequence as set forth in SEQ ID NO: 16 or SEQ ID NO: 33.

In the present application, the second binding domain may include an antibody heavy chain constant region.

In the present application, the antibody heavy chain constant region is derived from an IgG heavy chain constant region. For example, the antibody heavy chain constant region is derived from a human IgG heavy chain constant region. For example, the antibody heavy chain constant region is derived from a human IgG1 heavy chain constant region or a human IgG4 heavy chain constant region. For example, the antibody heavy chain constant region includes an amino acid sequence as set forth in SEQ ID NO: 43.

In the present application, the second binding domain may include an antibody heavy chain, and the antibody heavy chain may include an amino acid sequence as set forth in SEQ ID NO: 25 or SEQ ID NO: 41.

In the present application, the second binding domain may include at least one CDR in an antibody light chain variable region VL, and the VL includes an amino acid sequence as set forth in SEQ ID NO: 24 or SEQ ID NO: 40.

In the present application, the second binding domain may include LCDR3, and the LCDR3 may include an amino acid sequence as set forth in SEQ ID NO: 17 or SEQ ID NO: 34.

In the present application, the second binding domain may include LCDR2, and the LCDR2 may include an amino acid sequence as set forth in SEQ ID NO: 18 (WAS) or SEQ ID NO: 35 (LAS).

In the present application, the second binding domain may include LCDR1, and the LCDR1 may include an amino acid sequence as set forth in SEQ ID NO: 19 or SEQ ID NO: 36.

In the present application, the second binding domain may include LCDR1, LCDR2 and LCDR3, the LCDR1 may include an amino acid sequence as set forth in SEQ ID NO: 19 or SEQ ID NO: 36, the LCDR2 may include an amino acid sequence as set forth in SEQ ID NO: 18 (WAS) or SEQ ID NO: 35 (LAS), and the LCDR3 may include an amino acid sequence as set forth in SEQ ID NO: 17 or SEQ ID NO: 34. For example, the LCDR1 may include an amino acid sequence as set forth in SEQ ID NO: 19, the LCDR2 may include an amino acid sequence as set forth in SEQ ID NO: 18 (WAS), and the LCDR3 may include an amino acid sequence as set forth in SEQ ID NO: 17. For example, the LCDR1 may include an amino acid sequence as set forth in SEQ ID NO: 36, the LCDR2 may include an amino acid sequence as set forth in SEQ ID NO: 35 (LAS), and the LCDR3 may include an amino acid sequence as set forth in SEQ ID NO: 34.

In the present application, the second binding domain may include L-FR1, the C-terminus of the L-FR1 is directly or indirectly linked to the N-terminus of the LCDR1, and the L-FR1 may include an amino acid sequence as set forth in SEQ ID NO: 20 or SEQ ID NO: 37.

In the present application, the second binding domain may include L-FR2, the L-FR2 is between the LCDR1 and the LCDR2, and the L-FR2 may include an amino acid sequence as set forth in SEQ ID NO: 21 or SEQ ID NO: 38.

In the present application, the second binding domain may include L-FR3, the L-FR3 is between the LCDR2 and the LCDR3, and the L-FR3 may include an amino acid sequence as set forth in SEQ ID NO: 22 or SEQ ID NO: 39.

In the present application, the second binding domain may include L-FR4, the N-terminus of the L-FR4 is directly or indirectly linked to the C-terminus of the L-FR3, and the L-FR4 may include an amino acid sequence as set forth in SEQ ID NO: 23.

In the present application, the second binding domain may include L-FR1, L-FR2, L-FR3 and L-FR4, the L-FR1 may include an amino acid sequence as set forth in SEQ ID NO: 20 or SEQ ID NO: 37, the L-FR2 includes an amino acid sequence as set forth in SEQ ID NO: 21 or SEQ ID NO: 38, the L-FR3 may include an amino acid sequence as set forth in SEQ ID NO: 22 or SEQ ID NO: 39, and the L-FR4 may include an amino acid sequence as set forth in SEQ ID NO: 23. For example, the L-FR1 may include an amino acid sequence as set forth in SEQ ID NO: 20, the L-FR2 may include an amino acid sequence as set forth in SEQ ID NO: 21, the L-FR3 may include an amino acid sequence as set forth in SEQ ID NO: 22, and the L-FR4 may include an amino acid sequence as set forth in SEQ ID NO: 23. For example, the L-FR1 may include an amino acid sequence as set forth in SEQ ID NO: 37, the L-FR2 may include an amino acid sequence as set forth in SEQ ID NO: 38, the L-FR3 may include an amino acid sequence as set forth in SEQ ID NO: 39, and the L-FR4 may include an amino acid sequence as set forth in SEQ ID NO: 23.

In the present application, the second binding domain may include VL, and the VL may include an amino acid sequence as set forth in SEQ ID NO: 24 or SEQ ID NO: 40.

In the present application, the second binding domain may include an antibody light chain constant region. For example, the antibody light chain constant region is derived from an Igκ constant region. For example, the antibody light chain constant region is derived from a human Igκ constant region. For example, the antibody light chain constant region includes an amino acid sequence as set forth in SEQ ID NO: 44.

In the present application, the second binding domain may include an antibody light chain, and the antibody light chain may include an amino acid sequence as set forth in SEQ ID NO: 26 or SEQ ID NO: 42.

In the present application, the second binding domain may include VH and VL, the VH may include an amino acid sequence as set forth in SEQ ID NO: 16 or SEQ ID NO: 33, and the VL may include an amino acid sequence as set forth in SEQ ID NO: 24 or SEQ ID NO: 40. For example, the VH includes an amino acid sequence as set forth in SEQ ID NO: 16, and the VL includes an amino acid sequence as set forth in SEQ ID NO: 24. For example, the VH includes an amino acid sequence as set forth in SEQ ID NO: 33, and the VL includes an amino acid sequence as set forth in SEQ ID NO: 40.

In the present application, the second binding domain may include an antibody heavy chain and an antibody light chain. For example, the antibody heavy chain includes an amino acid sequence as set forth in SEQ ID NO: 25, and the antibody light chain includes an amino acid sequence as set forth in SEQ ID NO: 26. For example, the antibody heavy chain includes an amino acid sequence as set forth in SEQ ID NO: 41, and the antibody light chain includes an amino acid sequence as set forth in SEQ ID NO: 42.

### Antigen-binding protein capable of binding to CD39

In the present application, the antigen-binding protein capable of binding to CD39 may include an antibody or an antigen-binding fragment thereof.

In the present application, the antibody may be selected from the group consisting of a monoclonal antibody, a single-chain antibody, a chimeric antibody, a multispecific antibody, a humanized antibody, and a fully human antibody. In the present application, the antigen-binding fragment may be selected from the group consisting of: Fab, Fab', F(ab)₂, Fv, F(ab')₂, scFv, VHH, di-scFv and dAb fragments.

In the present application, the antigen-binding protein capable of binding to CD39 may be VHH.

In the present application, the antigen-binding protein capable of binding to CD39 of the isolated antigen-binding protein may include at least one CDR in an antibody heavy chain variable region VHH, and the VHH may include an amino acid sequence as set forth in SEQ ID NO: 8.

In the present application, the antigen-binding protein capable of binding to CD39 may include CDR3, and the CDR3 may include an amino acid sequence as set forth in SEQ ID NO: 1.

In the present application, the antigen-binding protein capable of binding to CD39 may include CDR2, and the CDR2 may include an amino acid sequence as set forth in SEQ ID NO: 2.

In the present application, the antigen-binding protein capable of binding to CD39 may include CDR1, and the CDR1 may include an amino acid sequence as set forth in SEQ ID NO: 3.

In the present application, the antigen-binding protein capable of binding to CD39 may include CDR1, CDR2 and CDR3, the CDR1 may include an amino acid sequence as set forth in SEQ ID NO: 3, the CDR2 may include an amino acid sequence as set forth in SEQ ID NO: 2, and the CDR3 may include an amino acid sequence as set forth in SEQ ID NO: 1.

In the present application, the antigen-binding protein capable of binding to CD39 may include FR1, the C-terminus of the FR1 is directly or indirectly linked to the N-terminus of the CDR1, and the FR1 may include an amino acid sequence as set forth in SEQ ID NO: 4.

In the present application, the antigen-binding protein capable of binding to CD39 may include FR2, the FR2 is between the CDR1 and the CDR2, and the FR2 may include an amino acid sequence as set forth in SEQ ID NO: 5.

In the present application, the antigen-binding protein capable of binding to CD39 may include FR3, the FR3 is between the CDR2 and the CDR3, and the FR3 may include an amino acid sequence as set forth in SEQ ID NO: 6.

In the present application, the antigen-binding protein capable of binding to CD39 may include FR4, the N-terminus of the FR4 is directly or indirectly linked to the C-terminus of the CDR3, and the FR4 may include an amino acid sequence as set forth in SEQ ID NO: 7.

In the present application, the antigen-binding protein capable of binding to CD39 may include VHH, and the VHH may include an amino acid sequence as set forth in SEQ ID NO: 8.

### Antigen-binding protein capable of binding to CD73

In the present application, the antigen-binding protein capable of binding to CD73 may include an antibody or an antigen-binding fragment thereof.

In the present application, the antibody may be selected from the group consisting of a monoclonal antibody, a single-chain antibody, a chimeric antibody, a multispecific antibody, a humanized antibody, and a fully human antibody.

In the present application, the antigen-binding fragment may be selected from the group consisting of: Fab, Fab', F(ab)₂, Fv, F(ab')₂, scFv, VHH, di-scFv and dAb fragments.

In the present application, the antigen-binding protein capable of binding to CD73 may include at least one CDR in an antibody heavy chain variable region VH, and the VH may include an amino acid sequence as set forth in SEQ ID NO: 16 or SEQ ID NO: 33.

In the present application, the antigen-binding protein capable of binding to CD73 may include HCDR3, and the HCDR3 may include an amino acid sequence as set forth in SEQ ID NO: 9 or SEQ ID NO: 27.

In the present application, the antigen-binding protein capable of binding to CD73 may include HCDR2, and the HCDR2 may include an amino acid sequence as set forth in SEQ ID NO: 10 or SEQ ID NO: 28.

In the present application, the antigen-binding protein capable of binding to CD73 may include HCDR1, and the HCDR1 may include an amino acid sequence as set forth in SEQ ID NO: 11 or SEQ ID NO: 29.

In the present application, the antigen-binding protein capable of binding to CD73 may include HCDR1, HCDR2 and HCDR3, the HCDR1 may include an amino acid sequence as set forth in SEQ ID NO: 11 or SEQ ID NO: 29, the HCDR2 may include an amino acid sequence as set forth in SEQ ID NO: 10 or SEQ ID NO: 28, and the HCDR3 may include an amino acid sequence as set forth in SEQ ID NO: 9 or SEQ ID NO: 27. For example, the HCDR1 may include an amino acid sequence as set forth in SEQ ID NO: 11, the HCDR2 may include an amino acid sequence as set forth in SEQ ID NO: 10, and the HCDR3 may include an amino acid sequence as set forth in SEQ ID NO: 9. For example, the HCDR1 may include an amino acid sequence as set forth in SEQ ID NO: 29, the HCDR2 may include an amino acid sequence as set forth in SEQ ID NO: 28, and the HCDR3 may include an amino acid sequence as set forth in SEQ ID NO: 27.

In the present application, the antigen-binding protein capable of binding to CD73 may include H-FR1, the C-terminus of the H-FR1 is directly or indirectly linked to the N-terminus of the HCDR1, and the H-FR1 may include an amino acid sequence as set forth in SEQ ID NO: 12 or SEQ ID NO: 30.

In the present application, the antigen-binding protein capable of binding to CD73 may include H-FR2, the H-FR2 is between the HCDR1 and the HCDR2, and the H-FR2 may include an amino acid sequence as set forth in SEQ ID NO: 13 or SEQ ID NO: 31.

In the present application, the antigen-binding protein capable of binding to CD73 may include H-FR3, the H-FR3 is between the HCDR2 and the HCDR3, and the H-FR3 may include an amino acid sequence as set forth in SEQ ID NO: 14 or SEQ ID NO: 32.

In the present application, the antigen-binding protein capable of binding to CD73 may include H-FR4, the N-terminus of the H-FR4 is directly or indirectly linked to the C-terminus of the HCDR3, and the H-FR4 may include an amino acid sequence as set forth in SEQ ID NO: 15.

In the present application, the antigen-binding protein capable of binding to CD73 may include H-FR1, H-FR2, H-FR3 and H-FR4, the H-FR1 may include an amino acid sequence as set forth in SEQ ID NO: 12 or SEQ ID NO: 30, the H-FR2 may include an amino acid sequence as set forth in SEQ ID NO: 13 or SEQ ID NO: 31, the H-FR3 may include an amino acid sequence as set forth in SEQ ID NO: 14 or SEQ ID NO: 32, and the H-FR4 may include an amino acid sequence as set forth in SEQ ID NO: 15. For example, the H-FR1 may include an amino acid sequence as set forth in SEQ ID NO: 12, the H-FR2 may include an amino acid sequence as set forth in SEQ ID NO: 13, the H-FR3 may include an amino acid sequence as set forth in SEQ ID NO: 14, and the H-FR4 may include an amino acid sequence as set forth in SEQ ID NO: 15. For example, the H-FR1 may include an amino acid sequence as set forth in SEQ ID NO: 30, the H-FR2 may include an amino acid sequence as set forth in SEQ ID NO: 31, the H-FR3 may include an amino acid sequence as set forth in SEQ ID NO: 32, and the H-FR4 may include an amino acid sequence as set forth in SEQ ID NO: 15.

In the present application, the antigen-binding protein capable of binding to CD73 may include VH, and the VH may include an amino acid sequence as set forth in SEQ ID NO: 16 or SEQ ID NO: 33.

In the present application, the antigen-binding protein capable of binding to CD73 may include an antibody heavy chain constant region.

In the present application, the antibody heavy chain constant region is derived from an IgG heavy chain constant region. For example, the antibody heavy chain constant region is derived from a human IgG heavy chain constant region. For example, the antibody heavy chain constant region is derived from a human IgG1 heavy chain constant region or a human IgG4 heavy chain constant region. For example, the antibody heavy chain constant region includes an amino acid sequence as set forth in SEQ ID NO: 43.

In the present application, the antigen-binding protein capable of binding to CD73 may include an antibody heavy chain, and the antibody heavy chain may include an amino acid sequence as set forth in SEQ ID NO: 25 or SEQ ID NO: 41.

In the present application, the antigen-binding protein capable of binding to CD73 may include at least one CDR in an antibody light chain variable region VL, and the VL includes an amino acid sequence as set forth in SEQ ID NO: 24 or SEQ ID NO: 40.

In the present application, the antigen-binding protein capable of binding to CD73 may include LCDR3, and the LCDR3 may include an amino acid sequence as set forth in SEQ ID NO: 17 or SEQ ID NO: 34.

In the present application, the antigen-binding protein capable of binding to CD73 may include LCDR2, and the LCDR2 may include an amino acid sequence as set forth in SEQ ID NO: 18 (WAS) or SEQ ID NO: 35 (LAS).

In the present application, the antigen-binding protein capable of binding to CD73 may include LCDR1, and the LCDR1 may include an amino acid sequence as set forth in SEQ ID NO: 19 or SEQ ID NO: 36.

In the present application, the antigen-binding protein capable of binding to CD73 may include LCDR1, LCDR2 and LCDR3, the LCDR1 may include an amino acid sequence as set forth in SEQ ID NO: 19 or SEQ ID NO: 36, the LCDR2 may include an amino acid sequence as set forth in SEQ ID NO: 18 (WAS) or SEQ ID NO: 35 (LAS), and the LCDR3 may include an amino acid sequence as set forth in SEQ ID NO: 17 or SEQ ID NO: 34. For example, the LCDR1 may include an amino acid sequence as set forth in SEQ ID NO: 19, the LCDR2 may include an amino acid sequence as set forth in SEQ ID NO: 18 (WAS), and the LCDR3 may include an amino acid sequence as set forth in SEQ ID NO: 17. For example, the LCDR1 may include an amino acid sequence as set forth in SEQ ID NO: 36, the LCDR2 may include an amino acid sequence as set forth in SEQ ID NO: 35 (LAS), and the LCDR3 may include an amino acid sequence as set forth in SEQ ID NO: 34.

In the present application, the antigen-binding protein capable of binding to CD73 may include L-FR1, the C-terminus of the L-FR1 is directly or indirectly linked to the N-terminus of the LCDR1, and the L-FR1 may include an amino acid sequence as set forth in SEQ ID NO: 20 or SEQ ID NO: 37.

In the present application, the antigen-binding protein capable of binding to CD73 may include L-FR2, the L-FR2 is between the LCDR1 and the LCDR2, and the L-FR2 may include an amino acid sequence as set forth in SEQ ID NO: 21 or SEQ ID NO: 38.

In the present application, the antigen-binding protein capable of binding to CD73 may include L-FR3, the L-FR3 is between the LCDR2 and the LCDR3, and the L-FR3 may include an amino acid sequence as set forth in SEQ ID NO: 22 or SEQ ID NO: 39.

In the present application, the antigen-binding protein capable of binding to CD73 may include L-FR4, the N-terminus of the L-FR4 is directly or indirectly linked to the C-terminus of the L-FR3, and the L-FR4 may include an amino acid sequence as set forth in SEQ ID NO: 23.

In the present application, the antigen-binding protein capable of binding to CD73 may include L-FR1, L-FR2, L-FR3 and L-FR4, the L-FR1 may include an amino acid sequence as set forth in SEQ ID NO: 20 or SEQ ID NO: 37, the L-FR2 may includes an amino acid sequence as set forth in SEQ ID NO: 21 or SEQ ID NO: 38, the L-FR3 may include an amino acid sequence as set forth in SEQ ID NO: 22 or SEQ ID NO: 39, and the L-FR4 may include an amino acid sequence as set forth in SEQ ID NO: 23. For example, the L-FR1 may include an amino acid sequence as set forth in SEQ ID NO: 20, the L-FR2 may include an amino acid sequence as set forth in SEQ ID NO: 21, the L-FR3 may include an amino acid sequence as set forth in SEQ ID NO: 22, and the L-FR4 may include an amino acid sequence as set forth in SEQ ID NO: 23. For example, the L-FR1 may include an amino acid sequence as set forth in SEQ ID NO: 37, the L-FR2 may include an amino acid sequence as set forth in SEQ ID NO: 38, the L-FR3 may include an amino acid sequence as set forth in SEQ ID NO: 39, and the L-FR4 may include an amino acid sequence as set forth in SEQ ID NO: 23.

In the present application, the antigen-binding protein capable of binding to CD73 may include VL, and the VL may include an amino acid sequence as set forth in SEQ ID NO: 24 or SEQ ID NO: 40.

In the present application, the antigen-binding protein capable of binding to CD73 may include an antibody light chain constant region. For example, the antibody light chain constant region is derived from an Igκ constant region. For example, the antibody light chain constant region is derived from a human Igκ constant region. For example, the antibody light chain constant region includes an amino acid sequence as set forth in SEQ ID NO: 44.

In the present application, the antigen-binding protein capable of binding to CD73 may may include an antibody light chain, and the antibody light chain may include an amino acid sequence as set forth in SEQ ID NO: 26 or SEQ ID NO: 42.

In the present application, the second binding domain may include VH and VL, the VH may include an amino acid sequence as set forth in SEQ ID NO: 16 or SEQ ID NO: 33, and the VL may include an amino acid sequence as set forth in SEQ ID NO: 24 or SEQ ID NO: 40. For example, the VH includes an amino acid sequence as set forth in SEQ ID NO: 16, and the VL includes an amino acid sequence as set forth in SEQ ID NO: 24. For example, the VH includes an amino acid sequence as set forth in SEQ ID NO: 33, and the VL includes an amino acid sequence as set forth in SEQ ID NO: 40.

In the present application, the second binding domain may include an antibody heavy chain and an antibody light chain. For example, the antibody heavy chain includes an amino acid sequence as set forth in SEQ ID NO: 25, and the antibody light chain includes an amino acid sequence as set forth in SEQ ID NO: 26. For example, the antibody heavy chain includes an amino acid sequence as set forth in SEQ ID NO: 41, and the antibody light chain includes an amino acid sequence as set forth in SEQ ID NO: 42.

### Nucleic acid molecule, vector, cell, and pharmaceutical composition

In another aspect, the present application provides an isolated nucleic acid molecule, which can encode the isolated antigen-binding protein as described in the present application. For example, the nucleic acid molecule may be produced or synthesized by: (i) amplification in vitro, e.g., by polymerase chain reaction (PCR); (ii) clonal recombination; (iii) purification, e.g., by enzymatic digestion and gel electrophoresis fractionation; or (iv) synthesis, e.g., chemical synthesis.

In another aspect, the present application provides a vector, which may include the nucleic acid molecule as described in the present application. In addition, the vector may further include other genes, for example, a marker gene that allows selection of the vector in an appropriate host cell and under an appropriate condition. In addition, the vector may further include an expression control element that allows correct expression of a coding region in an appropriate host. Such a control element is well known to those skilled in the art, and may include, for example, a promoter, ribosome binding site, enhancer, and other control elements that regulate gene transcription or mRNA translation. The vector may be transformed, transduced, or transfected into a host cell to express a genetic material element it carries within the host cell. The vector may include, for example, a plasmid, cosmid, virus, phage, or other vectors commonly used in, for example, genetic engineering. For example, the vector is an expression vector. In addition, the vector may also include a component that facilitates entry into a cell, e.g., a viral particle, a liposome, or a protein coat, which are not limited thereto.

In another aspect, the present application provides a cell, which may include the nucleic acid molecule as described in the present application, and/or the vector as described in the present application. In some embodiments, each type of or each host cell includes one or one type of the nucleic acid molecules or the vectors as described in the present application. In some embodiments, each type of or each host cell includes multiple (e.g., 2 or more) or multiple types (e.g., 2 or more types) of the nucleic acid molecules or the vectors as described in the present application. For example, the vector as described in the present application may be introduced into the host cell, e.g., a eukaryotic cell, such as a plant-derived cell, a fungus, or a yeast cell. In some embodiments, the cell is a bacterial cell (e.g., E. coli), a yeast cell, or any other eukaryotic cell, e.g., a COS cell, a Chinese hamster ovary (CHO) cell, a CHO-K1 cell, a LNCAP cell, a HeLa cell, a 293T cell, a COS-1 cell, a SP2/0 cell, a NS0 cell, or a myeloma cell. The vector as described in the present application may be introduced into the host cell by methods known in the art, for example, electroporation, lipofectine transfection, and lipofectamin transfection.

In another aspect, the present application further provides a pharmaceutical composition, which may include the isolated antigen-binding protein as described in the present application, the nucleic acid molecule as described in the present application, the vector as described in the present application, and/or the cell as described in the present application, and optionally a pharmaceutically acceptable carrier.

In the present application, the pharmaceutical composition may also include one or more suitable preparations of a (pharmaceutically effective) adjuvant, a stabilizer, a excipient, a diluent, a solubilizer, a surfactant, a emulsifier and/or a preservative. The acceptable components of the composition are preferably non-toxic to a recipient at the dose and concentration used. The pharmaceutical composition of the present application includes, but is not limited to, a liquid, frozen or freeze-dried composition.

In the present application, the pharmaceutical composition may also include one or more than one active compound, typically those active compounds which have complementary activity and do not adversely affect one another. The type and effective dosage of such drugs may depend on factors such as the amount and type of an antagonist present in a preparation, as well as clinical parameters of a subject.

In the present application, the pharmaceutically acceptable carrier may include any and all solvents, dispersion media, coatings, isotonic agents, and absorption delay agents that are compatible with drug administration, typically safe and non-toxic.

In the present application, the pharmaceutical composition may be administered parenterally, percutaneously, intracavitary, intra-arterially, intrathecally, and/or intranasally, or injected directly into tissues. For example, the pharmaceutical composition may be administered to patients or subjects by infusion or injection. **In** some embodiments, the administration of the pharmaceutical composition can be performed in various ways, such as intravenous, intraperitoneal, subcutaneous, intramuscular, topical, or intradermal administration. **In** some embodiments, the pharmaceutical composition can be administered uninterruptedly. The uninterrupted (or continuous) administration may be achieved through a small pump system worn by a patient to measure the therapeutic agent flowing into the patient's body, as described in WO2015/036583.

### Preparation method

In another aspect, the present application provides a method for preparing the antigen-binding protein. The method may include: the host cell as described in the present application is cultured under a condition that allows expression of the isolated antigen-binding protein. For example, by using appropriate culture media, appropriate temperature, and culture time, these methods are known methods to those skilled in the art.

### Method and use

In another aspect, the present application provides use of the isolated antigen-binding protein, the pharmaceutical combination, the nucleic acid molecule, the vector, the cell, and/or the pharmaceutical composition in preparation of a drug, wherein the drug is used for preventing and/or treating diseases and/or disorders.

In another aspect, the present application further provides a method for preventing and/or treating diseases and/or disorders, and the method may include: the isolated antigen-binding protein, the pharmaceutical combination, the nucleic acid molecule, the vector, the cell, and/or the pharmaceutical composition as described in the present application is administered to subjects in need thereof.

In the present application, the administration may be carried out in different ways, such as intravenous, intratumoral, intraperitoneal, subcutaneous, intramuscular, topical or intradermal administration.

In another aspect, the isolated antigen-binding protein, the pharmaceutical combination, the nucleic acid molecule, the vector, the cell, and/or the pharmaceutical composition as described in the present application may be used for preventing and/or treating diseases and/or disorders.

In the present application, the diseases and/or disorders may be mediated by CD39 and/or CD73.

In the present application, the diseases and/or disorders may include tumors.

In the present application, the tumors may include solid tumors and/or hematologic tumors.

In the present application, the diseases and/or disorders may include pancreatic cancer and/or colorectal cancer.

In another aspect, the present application further provides a method for detecting CD39 and/or CD73 in a sample, and the method includes application of the isolated antigen-binding protein, the pharmaceutical combination, the nucleic acid molecule, the vector, the cell, and/or the pharmaceutical composition.

In the present application, the method for detecting CD39 and/or CD73 in a sample may be an in vitro method. In the present application, the method for detecting CD39 and/or CD73 in a sample can be used for non-therapeutic purposes. In the present application, the method for detecting CD39 and/or CD73 in a sample is not a diagnostic method.

In another aspect, the present application further provides a reagent or a kit for detecting CD39 and/or CD73 in a sample, and the reagent or the kit includes the isolated antigen-binding protein, the pharmaceutical combination, the nucleic acid molecule, the vector, the cell, and/or the pharmaceutical composition.

In another aspect, the present application further provides use of the isolated antigen-binding protein, the pharmaceutical combination, the nucleic acid molecule, the vector, the cell, and/or the pharmaceutical composition in preparation of a kit, and the kit is used for detecting the presence and/or content of CD39 and/or CD73 in a sample.

Without intending to be limited by any theory, the examples described below are only intended to illustrate the antigen-binding protein, preparation method, and uses of the present application, and are not intended to limit the scope of the present invention in the application.

### Examples

### Example 1 Sequence design of heavy chain and light chain of CD39-CD73 bispecific antibody

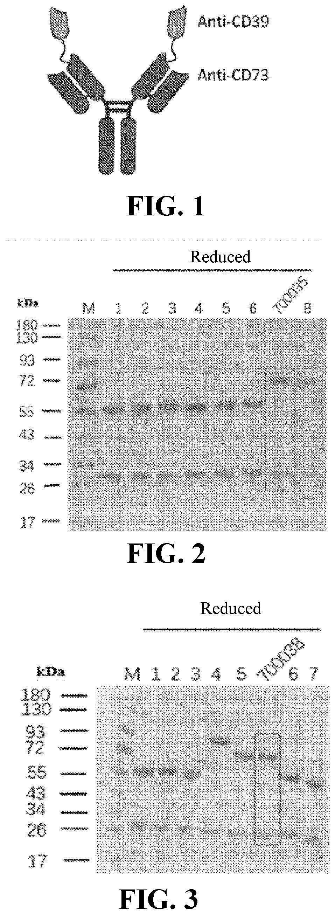

The structure of the CD39-CD73 bispecific antibody in the present application belongs to VHH-IgG, which means that the N-termini of both heavy chains of one IgG antibody are all linked to a VHH fragment of another nanobody, the configuration of which is shown in FIG. 1. This form of bispecific antibody structure is abbreviated as CD39-VHH-CD73-IgG, the main composition design of heavy chain and light chain of which is as shown in Table 1 below.

**Table 1 Main composition design of CD39-VHH-CD73-IgG heavy chain and light chain**

| Bispecific antibody number | Heavy chain | | | Light chain |
|---|---|---|---|---|
| | VHH moiety | Linker fragment | Heavy chain variable region of IgG moiety | |
| 700035 | 700053 (SEQ ID NO: 8) | Linker 1 (SEQ ID NO: 47) | 900725-VH (SEQ ID NO: 33) | 900725-VL (SEQ ID NO: 40) |
| 700038 | 700053 (SEQ ID NO: 8) | Linker1 (SEQ ID NO: 47) | 900698-VH (SEQ ID NO: 16) | 900698-VL (SEQ ID NO: 24) |

The heavy chain sequences of bispecific antibodies 700035 and 700038 in the form of CD39-VHH-CD73-IgG are shown in Table 2.

**Table 2 Heavy chain sequence of CD39-VHH-CD73-IgG bispecific antibody**

| Protein number | Heavy chain sequence |
|---|---|
| 700035 | SEQ ID NO: 45 |
| 700038 | SEQ ID NO: 46 |

The light chain sequences of CD39-VHH-CD73-IgG format bispecific antibodies 700035 and 700038 are derived from the light chains of 900725 (SEQ ID NO: 42) and 900698 (SEQ ID NO: 26), respectively.

### Example 2 Expression and purification of CD39-CD73 bispecific antibody

An expression vector obtained in Example 1 was amplified by E. coli and a sufficient number of plasmids were prepared using an endotoxin-free plasmid extraction kit (Tiangen Biochemical Technology (Beijing) Co., Ltd., #DP117) for transient transfection and expression of bispecific antibodies. The host cells used for expression were CHO-S cells (Thermo Fisher, #R80007). By mixing the prepared two heavy chain vectors and the light chain vectors with polyetherimide (PEI, Polysciences, #24765-1), liposome complexes were formed to transfect CHO-S cells, and then the transfected cells were cultured in an incubator for 5-7 days. The supernatant of the cell culture was collected by centrifugation and purified through a Protein A affinity chromatography column to obtain bispecific antibodies, and further the aggregation of proteins was determined by molecular exclusion chromatography.

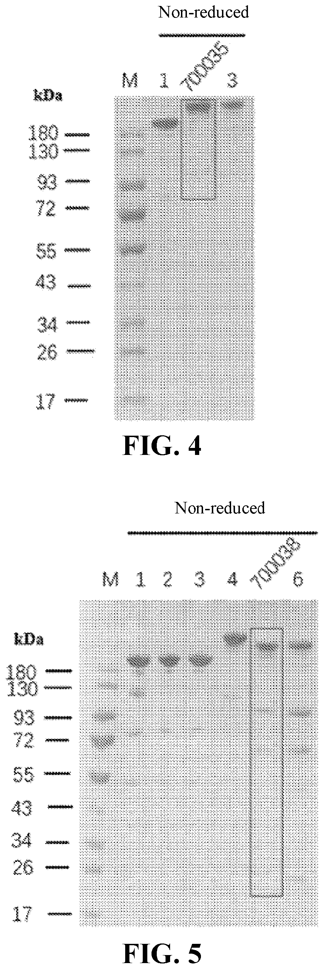

The expression, purification and detection of the bispecific antibodies are shown in FIG. 2 to FIG. 5 and Table 3. The detection results indicate that for the bispecific antibodies in the form of CD39-VHH-CD73-IgG, the SEC purities of IgGs derived from different parental monoclonal antibodies are better, reaching about 95%. The reduced SDS-PAGE images show that neither the heavy nor light chains of 700035 and 700038 were degraded, indicating excellent stability of 700035 and 700038.

**Table 3 SEC purity data of CD39-CD73 bispecific antibody**

| Antibody number | 700035 | 700038 |
|---|---|---|
| SEC-HPLC purity (%) | 95.54 | 96.46 |

### Example 3 Determination of kinetic parameters of CD39-CD73 bispecific antibody

The binding kinetics and affinity of CD39-CD73 bispecific antibodies 700035 and 700038, as well as parental control antibodies 700053 (CD39 nanobody), 900698 (CD73 parental monoclonal antibody corresponding to 700038), and 900725 (CD73 parental monoclonal antibody corresponding to 700035) to antigens were determined by an SPR method in the test.

### The test method is as follows:

An Anti-Human Capture-CM5 chip (GE, #BR-1005-30) was prepared using coupling methods of a Human Antibody Capture Kit (GE, #BR-1008-39) and an amino coupling kit (GE, BR-1000-50). The chip was equilibrated at room temperature for 20-30 mins and loaded into a Biacore 8K instrument. The antigen and antibody were diluted to an experimental working concentration using an equilibration buffer. The antigens were diluted with the equilibrium buffer to 50 nM, and then diluted three-fold for seven concentration gradients, and two zero concentrations (i.e., the equilibrium buffer) and one repeat concentration (typically the lowest concentration repeat) were set. Experimental analysis was performed on 10 antigen concentrations (2 zero concentrations, 7 gradient concentrations, and 1 repeated concentration) in a cyclic manner according to an order of antibody, antigen, and regeneration. The antigen injection flow rate was 30 µL/min, the binding time was 120 s, and the dissociation time was 600 s. Following the analysis, the data was analyzed using the corresponding analysis program to confirm that there was no obvious reference binding. Using Kinetics and a 1:1 binding model, the data was fit to obtain the kinetic related parameters Ka, Kd, and KD values of the bispecific antibodies and parental control antibodies.

The detection results are shown in Tables 4 and 5, which indicate that the affinities of 700035 and 700038 for the human CD39 antigen are comparable and significantly better than those of the parental nanobody 700053 for the CD39 antigen. For the human CD73 antigen, the affinity of the 700035 bispecific antibody is comparable to that of the parental 700025 monoclonal antibody, and the affinity of the 700038 bispecific antibody is comparable to that of the 900698 monoclonal antibody for the CD73 antigen.

**Table 4 Detection of binding affinity of candidate antibodies to human CD39 antigen**

| Ligand | Analyte | ka (1/Ms) | kd (1/s) | KD (M) |
|---|---|---|---|---|
| 700053 | Human CD39 | 7.30E+05 | 1.02E-04 | 1.40E-10 |
| 700035 | Human CD39 | 1.31E+06 | 9.28E-05 | 7.08E-11 |
| 700038 | Human CD39 | 1.04E+06 | 9.54E-05 | 9.18E-11 |

**Table 5 Detection of binding affinity of candidate antibodies toCD73 antigen**

| Ligand | Analyte | ka (1/Ms) | kd (1/s) | KD (M) |
|---|---|---|---|---|
| 900698 | Human CD73 | 5.61E+05 | 2.15E-04 | 3.82E-10 |
| 900725 | Human CD73 | 9.88E+05 | 1.82E-04 | 1.84E-10 |
| 700035 | Human CD73 | 9.35E+05 | 3.19E-04 | 3.41E-10 |
| 700038 | Human CD73 | 5.87E+05 | 2.49E-04 | 4.25E-10 |

### Example 4 Determination of biological activity of CD39-CD73 bispecific antibody

### 4.1 Inhibitory effect of CD39-CD73 bispecific antibody on CD39 function

The experimental steps for detecting the inhibitory effect of the CD39-CD73 bispecific antibody on the CD39 function are as follows: the antibody was diluted with TM buffer working solution (TM buffer stock solution mixed with DI Water at a ratio of 1:1) to 30 µg/mL, and then diluted three-fold for 10 gradients. CHOK1-huCD39-10B6 cells overexpressing CD39 were counted, resuspended in TM buffer working solution to 1*10 E5/mL, and seeded at 50 µL per well into a 96 well U-plate. The diluted antibody was added at 50 µL per well to the 96 well U-plate with the pre-seeded cells and incubated for 1 h. ATP was diluted to 60 µM with TM Buffer working solution, added at 50 µL per well to the 96 well U-plate after the cell-antibody incubation, and incubated for 1 h. Following the incubation, the culture plate was centrifuged at 400 G for 2 min, and 50 µL of supernatant was transferred from each well to the corresponding well in a 96 well black plate. CellTiter Glo^{®} Substrate was added to CellTiter Glo^{®} buffer, mixed well, equilibrated to room temperature, then added at 50 µL per well to the 96 well black plate, and reacted in the dark at room temperature for 10 min. Finally, the signal intensity was measured using an MD i3x enzyme-linked immunosorbent assay reader.

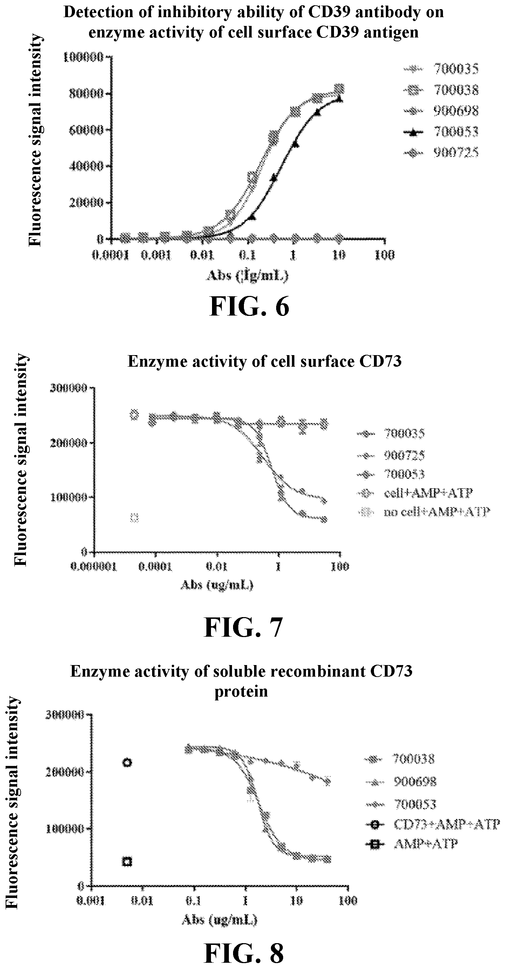

The detection results are shown in FIG. 6 and Table 6. The results indicate that the inhibitory abilities of the bispecific antibodies 700035 and 700038 on the enzyme activity of the CD39 antigen are about three times stronger than that of the parental CD39 nanoantibody 700053, and the inhibitory abilities of the two bispecific antibodies are comparable.

**Table 6 Inhibitory effects of bispecific antibodies 700035 and 700038 on CD39 function**

| Antibody | 700053 | 700035 | 700038 |
|---|---|---|---|
| EC50 (µg/mL) | 0.5576 | 0.2082 | 0.1871 |

### 4.2 Inhibitory effect of CD39-CD73 bispecific antibody on CD73 function

4.2.1 The experimental steps for detecting the inhibitory effect of the CD39-CD73 bispecific antibody 700035 on the enzyme activity function of membrane surface CD73 are as follows:
The antibody was diluted with PBS to 30 µg/mL, and then diluted 5-fold for 8 gradients. CHOK1-huCD73-3F4 cells overexpressing CD73 were counted, resuspended in a culture medium at 4*10 E5/mL, seeded at 100 µL per well into a 96 well U-plate, and centrifuged at1500 rpm for 5 min, and the supernatant was discarded. The cells were resuspended with the diluted series of antibodies at 100 µL per well, and incubated at 37°C for 20 min. Following the incubation, each well was washed with 200 µL of TM Buffer working solution (TM buffer stock solution mixed with DI Water at a 1:1 ratio), and centrifuged at 1500 rpm for 5 min, and the supernatant was discarded. Then each well was washed with 200 µL of TM Buffer working solution and centrifuged again, and the supernatant was discarded. AMP was diluted with TM Buffer working solution to 180 µM, added for resuspending the cells in the above culture plate at 100 µL per well, and incubated at 37°C for 1 h. Following incubation, the culture plate was centrifuged at 1500 rpm for 5 min, and 50 µL of supernatant was transferred from each well to the corresponding well in a 96 well black plate. ATP was diluted with TM Buffer working solution to 60 µM, added at 50 µL per well to the 96 well black plate, and incubated at 37°C for 15 min. CellTiter Glo^{®} Substrate was added to CellTiter Glo^{®} buffer, mixed well, equilibrated to room temperature, then added at 100 µL per well to the 96 well black plate, and reacted in the dark at room temperature for 10 min. Finally, the signal intensity was measured using an MD i3x enzyme-linked immunosorbent assay reader.

The detection results of the bispecific antibody are shown in FIG. 7 and Table 7. The results indicate that the inhibitory effect of the bispecific antibody 700035 on the CD73 function weakens only about twice compared to the CD73 parental monoclonal antibody 900725.

**Table 7 Inhibitory effect of bispecific antibody 700035 on enzyme activity function of membrane expressing CD73**

| Antibody | 900725 | 700035 |
|---|---|---|
| EC50 (µg/mL) | 0.32 | 0.6147 |

4.2.2 The experimental steps for detecting the inhibitory effect of the CD39-CD73 bispecific antibody 700038 on the enzymatic activity function of the soluble recombinant protein CD73 are as follows:
The antibody was diluted with TM buffer working solution (TM buffer stock solution mixed with DI Water at a 1:1 ratio) to 128 µg/mL, and then diluted 2-fold for 10 gradients. The CD73 soluble protein was diluted with TM buffer working solution to 0.3 µg/mL, and then the diluted bispecific antibody (25 µL per well) and the CD73 soluble protein (25 µL per well) were added to a 96 well black plate in sequence. AMP and ATP were diluted with TM Buffer working solution to 240 µM and 80 µM respectively and mixed at a 1:1 ratio. Then 50 µL of the resulting mixture was added to each well of the 96 well black plate containing the antibody-soluble CD73 protein, and reacted for 10 min. CellTiter Glo^{®} Substrate was added to CellTiter Glo^{®} buffer, mixed well, equilibrated to room temperature, then added at 100 µL per well to the 96 well black plate, and reacted in the dark at room temperature for 10 min. Finally, the signal intensity was measured using an MD i3x enzyme-linked immunosorbent assay reader.

The detection results of the bispecific antibody are shown in FIG. 8 and Table 8. The results indicate that the inhibitory effect of the bispecific antibody 700038 on the CD73 function is similar to that of the CD73 parental monoclonal antibody 900698.

**Table 8 Inhibitory effect of bispecific antibody 700038 on enzyme activity function of soluble protein CD73**

| Antibody | 900698 | 700038 |
|---|---|---|
| EC50 (µg/mL) | 1.742 | 1.849 |

### Example 5 Detection of thermal stability of CD39-CD73 bispecific antibody

The experimental steps for detecting the thermal stability of the CD39-CD73 bispecific antibodies 700035 and 700038 are as follows: the antibody was concentrated to 20 mg/mL, wherein a buffer system is 20 mM acetic acid-sodium acetate and 8% w/v sucrose, pH 5.7. The melting temperature (Tm) and aggregation temperature (Tagg) of the bispecific antibodies 700035 and 700038 were detected using a protein stability analyzer (UNcle, UNCHAINED LABS, US), wherein the temperature rise range for Tm and Tagg was 25°C to 95°C, with a heating rate of 0.3°C.

The detection results of the thermal stability of the bispecific antibodies are shown in Table 9. The results indicate that the thermal stability data Tm and Tagg values of such form of bispecific antibodies are both in the range of 69°C to 80°C, exhibiting excellent thermal stability performance, among which the thermal stability of 700035 is better than that of 700038.

**Table 9 Thermal stability data of bispecific antibodies 700035 and 700038**

| Antibody | Tm value (°C) | Tagg value (°C) |
|---|---|---|
| 700035 | 70.1 | 78.5 |
| 700038 | 69.8 | 74.4 |

### Example 6 Species cross test of CD39-CD73 bispecific antibody

The binding kinetics and affinities of CD39-CD73 bispecific antibodies 700035 and 700038 to CD39&CD73 antigens from different species (Human/Cynomolgus/Mouse) were determined by an SPR method in the test. The experimental method is as follows:
An Anti-Human Capture-CM5 chip (GE, #BR-1005-30) was prepared using coupling methods of a Human Antibody Capture Kit (GE, #BR-1008-39) and an amino coupling kit (GE, BR-1000-50). The chip was equilibrated at room temperature for about 20-30 min and loaded into a Biacore 8K instrument. The antigen and antibody were diluted to an experimental working concentration using an equilibration buffer. The antigen was diluted with an equilibrium buffer to 50 nM, and then diluted three-fold for seven concentration gradients, and two zero concentrations (i.e., the equilibrium buffer) and one repeat concentration (typically the lowest concentration repeat) were set. Experimental analysis was performed on 10 antigen concentrations (2 zero concentrations, 7 gradient concentrations, and 1 repeated concentration) in a cyclic manner according to an order of antibody, antigen, and regeneration. The antigen injection flow rate was 30 µL/min, the binding time was 120 s, and the dissociation time was 600 s. Following the analysis, the data was analyzed using the corresponding analysis program to confirm that there was no obvious reference binding. Using Kinetics and a 1:1 binding model, the data was fit to obtain the kinetic related parameters Ka, Kd, and KD values of the bispecific antibodies.

The test results are shown in Table 10. The results indicate that for the CD39 antigen, the two bispecific antibodies 700035 and 700038 have extremely high affinity for the human CD39 antigen, reaching 7.08E-11 and 9.18E-11 respectively. The affinity for the cynomolgus CD39 antigen also reaches 2.61E-9 and 2.69E-9 respectively. For the CD73 antigen, the affinities of the two bispecific antibodies 700035 and 700038 for the human CD73 antigen and cynomolgus CD73 antigen are comparable, reaching about E-10. In addition, both the bispecific antibodies did not bind to the mouse CD39 and CD73. In summary, for the CD39 and CD73 targets, humans and cynomolgus monkeys are related species, while mice are not related species.

**Table 10 Detection of affinity for CD39 and CD73 antigens from different species**

| Ligand | Analyte | ka (1/Ms) | kd (1/s) | KD (M) |
|---|---|---|---|---|
| 700035 | Human CD39 | 1.31E+06 | 9.28E-05 | 7.08E-11 |
| | Cynomolgus CD39 | 6.18E+05 | 1.61E-03 | 2.61E-9 |
| | Mouse CD39 | / | / | / |
| | Human CD73 | 9.35E+05 | 3.19E-04 | 3.41E-10 |
| | Cynomolgus CD73 | 8.36E+05 | 3.42E-04 | 4.09E-10 |
| | Mouse CD73 | / | / | / |
| 700038 | Human CD39 | 1.04E+06 | 9.54E-05 | 9.18E-11 |
| | Cynomolgus CD39 | 6.06E+05 | 1.63E-03 | 2.69E-09 |
| | Mouse CD39 | / | / | / |
| | Human CD73 | 5.87E+05 | 2.49E-04 | 4.25E-10 |
| | Cynomolgus CD73 | 4.56E+05 | 1.48E-04 | 3.26E-10 |
| | Mouse CD73 | / | / | / |

### Example 7 Pharmacodynamic study of test substance in human pancreatic cancer cell BxPC-3 hPBMC model

Human pancreatic cancer cells BxPC-3 were cultured in an RPMI-1640 medium supplemented with 10% FBS in a 37°C incubator containing 5% CO₂. Before the cells were continuously cultured ten generations, 100 µL of PBS containing about 1×10⁷ BxPC-3 cells and an equal volume of Matrigel were mixed well and subcutaneously injected into the right back of 30 NPG mice near the axilla, with an inoculation volume of about 200 µL. The mice were anesthetized with 2-5% isoflurane before the inoculation. When the tumor grew to an average of about 50-80 mm³, the 30 tumor bearing mice were randomly divided into 5 groups based on the tumor volume and body weight, with 6 mice in each group, and injected with 1×10⁷ PBMCs (100 µL) via the tail vein. On the day of grouping, administration was carried out, and the administration volume of the animals was adjusted according to 10 µL/g body weight. The grouping situation and dosing regimen are shown in Table 10. The appearance and behavior of each mouse were observed daily from the day of grouping continuously for 4 weeks. The body weight (BW), tumor volume (TV), relative tumor volume (RTV), and relative tumor growth inhibition (TGI) of the animals were measured.

**Table 11 Grouping and dosing regimen**

| **Group** | **Number of mice** | **Test substance** | **Dose (mg/kg)** | **Dosing regimen** |
|---|---|---|---|---|
| **1** | 6 | 900543 | 5 | i.p. BIW*4W |
| **2** | 6 | 900725 | 5 | i.p. BIW*4W |
| **3** | 6 | 700053 | 2.7 | i.p. BIW*4W |
| **4** | 6 | 900725+700053 | 5+2.7 | i.p. BIW*4W |
| **5** | 6 | 700035 | 5.9 | i.p. BIW*4W |

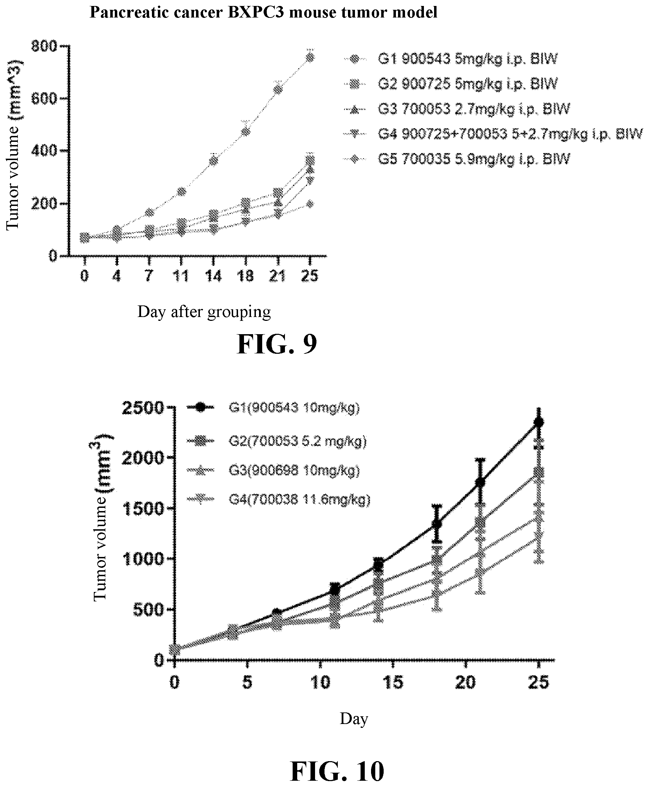

The results are shown in FIG. 9. From the results, compared with the negative control antibody 900543 (the heavy chain sequence is set forth in SEQ ID NO: 48, and the light chain sequence is set forth in SEQ ID NO: 49), both the CD39 nanoantibody 700053 and the CD73 monoclonal antibody 900725 have good inhibitory effects on pancreatic cancer tumor growth. The combination of the 700053 and 900725 has a better anti-tumor effect compared to single use of the two monoclonal antibodies. Also, the CD39/CD73 bispecific antibody 700035 showed a comparable effect to the combination of the 700053 and 9000725, demonstrating a synergistic anti-tumor effect of the bispecific antibody compared to the two parental antibodies.

### Example 8 Pharmacodynamic study of test substance in mouse colorectal cancer model

MC38-hCD73 mouse colorectal cancer tumor cells were injected subcutaneously on the right side of female B-hCD73/hCD39 (v3) humanized mice at a concentration of 5×10⁵ cells/0.1 mL. When the tumor grew to about 101 mm³, the mice were randomly divided into groups for administration (the day of grouping was denoted as D0). The grouping and dosing regimen are shown in Table 11. Each group consisted of 6 mice, with a total of 4 groups, namely 900543 (hIgG1 isotype control, 10 mg/kg), 700053 (CD39 nanomonoclonal antibody, 5.2 mg/kg), 900698 (CD73 monoclonal antibody, 10 mg/kg), and 700038 (CD39/CD73 bispecific antibody, 11.6 mg/kg). After grouping, each group of mice was administered intraperitoneal injection twice a week for 4 consecutive weeks, for a total of 8 times. The body weight and tumor volume of the mice were measured twice a week during administration and observation, and the measurement values were recorded. On the 25th day (D25) after grouping, the experiment was ended, and the animals were euthanized. The tumors were removed, weighed, and photographed. The tumor growth inhibition on tumor volume TGITV (%) and tumor growth inhibition on tumor weight TGITW (%) were calculated and statistically analyzed. During the treatment period, all groups of mice had normal food and water intake, stable body weight, no cessation of medication, no death, no significant toxic side effects observed, and good tolerance.

**Table 12 Grouping and dosing regimen**

| **Group** | **Number of mice** | **Test substance** | **Dose (mg/kg)** | **Dosing regimen** |
|---|---|---|---|---|
| **1** | 6 | 900543 | 10 | i.p. BIW*4W |
| **2** | 6 | 700053 | 5.2 | i.p. BIW*4W |
| **3** | 6 | 900698 | 10 | i.p. BIW*4W |
| **4** | 6 | 700038 | 11.6 | i.p. BIW*4W |

As shown in FIG. 10, at the end of the experiment (D25), the average tumor volumes of the 700053 (5.2 mg/kg) group, the 900698 (10 mg/kg) group, and the 700038 (11.6 mg/kg) group were 1858±318 mm3, 1420±347 mm3, and 1213±274 mm3 respectively, with corresponding tumor growth inhibition on tumor volume TGITV of 21.9%, 41.4%, and 50.6%. Compared to the isotype control antibody 900543 group (2352±248 mm3&2.477±0.358 g), the tumor volume and weight of the 700053, 900698, and 700038 groups were significantly reduced and decreased. The data indicate that the 700053, 900698, and 700038 all have inhibitory effects on tumor growth, and the CD39/CD73 bispecific anti-tumor effect has a synergistic effect compared to the CD39 nanomonoclonal antibody and the CD73 monoclonal antibody.

## Claims

1. An isolated antigen-binding protein, comprising a first binding domain and a second binding domain, wherein said first binding domain is capable of binding to a CD39 protein, and said second binding domain is capable of binding to a CD73 protein.

2. The isolated antigen-binding protein of claim 1, having one or more of the following properties:
(1) capability of binding to a CD39 protein at a K_{D} value of 1×10⁻⁹ M or lower;
(2) capability of binding to a CD73 protein at a K_{D} value of 1×10⁻⁹ M or lower;
(3) inhibitory effect on enzymatic activity of a CD39 antigen;
(4) inhibitory effect on enzymatic activity of a CD73 antigen;
(5) thermal stability;
(6) capability of binding to human CD39 and/or cynomolgus CD73;
(7) capability of binding to human CD73 and/or cynomolgus CD73; and
(8) capability of inhibiting growth of tumor cells.

3. The isolated antigen-binding protein of any one of claims 1 to 2, wherein said first binding domain comprises an antibody or an antigen-binding fragment thereof.

4. The isolated antigen-binding protein of claim 3, wherein said antibody is selected from the group consisting of a monoclonal antibody, a single-chain antibody, a chimeric antibody, a multispecific antibody, a humanized antibody, and a fully human antibody.

5. The isolated antigen-binding protein of any one of claims 3 to 4, wherein said antigen-binding fragment is selected from the group consisting of Fab, Fab', F(ab)₂, Fv, F(ab')₂, scFv, VHH, di-scFv and dAb fragments.

6. The isolated antigen-binding protein of any one of claims 1 to 5, wherein said first binding domain is VHH.

7. The isolated antigen-binding protein of any one of claims 1 to 6, wherein said first binding domain comprises at least one CDR in an antibody heavy chain variable region VHH, and said VHH comprises an amino acid sequence as set forth in SEQ ID NO: 8.

8. The isolated antigen-binding protein of any one of claims 1 to 7, wherein said first binding domain comprises CDR3, and said CDR3 comprises an amino acid sequence as set forth in SEQ ID NO: 1.

9. The isolated antigen-binding protein of any one of claims 1 to 8, wherein said first binding domain comprises CDR2, and said CDR2 comprises an amino acid sequence as set forth in SEQ ID NO: 2.

10. The isolated antigen-binding protein of any one of claims 1 to 9, wherein said first binding domain comprises CDR1, and said CDR1 comprises an amino acid sequence as set forth in SEQ ID NO: 3.

11. The isolated antigen-binding protein of any one of claims 1 to 10, wherein said first binding domain comprises FR1, the C-terminus of said FR1 is directly or indirectly linked to the N-terminus of said CDR1, and said FR1 comprises an amino acid sequence as set forth in SEQ ID NO: 4.

12. The isolated antigen-binding protein of any one of claims 1 to 11, wherein said first binding domain comprises FR2, said FR2 is between said CDR1 and said CDR2, and said FR2 comprises an amino acid sequence as set forth in SEQ ID NO: 5.

13. The isolated antigen-binding protein of any one of claims 1 to 12, wherein said first binding domain comprises FR3, said FR3 is between said CDR2 and said CDR3, and said FR3 comprises an amino acid sequence as set forth in SEQ ID NO: 6.

14. The isolated antigen-binding protein of any one of claims 1 to 13, wherein said first binding domain comprises FR4, the N-terminus of said FR4 is directly or indirectly linked to the C-terminus of said CDR3, and said FR4 comprises an amino acid sequence as set forth in SEQ ID NO: 7.

15. The isolated antigen-binding protein of any one of claims 1 to 14, wherein said first binding domain comprises VHH, and said VHH comprises an amino acid sequence as set forth in SEQ ID NO: 8.

16. The isolated antigen-binding protein of any one of claims 1 to 15, wherein said second binding domain comprises an antibody or an antigen-binding fragment thereof.

17. The isolated antigen-binding protein of claim 16, wherein said antibody is selected from the group consisting of a monoclonal antibody, a single-chain antibody, a chimeric antibody, a multispecific antibody, a humanized antibody, and a fully human antibody.

18. The isolated antigen-binding protein of any one of claims 16 to 17, wherein said antigen-binding fragment is selected from the group consisting of Fab, Fab', F(ab)₂, Fv, F(ab')₂, scFv, VHH, di-scFv and dAb fragments.

19. The isolated antigen-binding protein of any one of claims 1 to 18, wherein said second binding domain comprises at least one CDR in an antibody heavy chain variable region VH, and said VH comprises an amino acid sequence as set forth in SEQ ID NO: 16 or SEQ ID NO: 33.

20. The isolated antigen-binding protein of any one of claims 1 to 19, wherein said second binding domain comprises HCDR3, and said HCDR3 comprises an amino acid sequence as set forth in SEQ ID NO: 9 or SEQ ID NO: 27.

21. The isolated antigen-binding protein of any one of claims 1 to 20, wherein said second binding domain comprises HCDR2, and said HCDR2 comprises an amino acid sequence as set forth in SEQ ID NO: 10 or SEQ ID NO: 28.

22. The isolated antigen-binding protein of any one of claims 1 to 21, wherein said second binding domain comprises HCDR1, and said HCDR1 comprises an amino acid sequence as set forth in SEQ ID NO: 11 or SEQ ID NO: 29.

23. The isolated antigen-binding protein of any one of claims 1 to 22, wherein said second binding domain comprises HCDR1, HCDR2 and HCDR3, and said HCDR1, HCDR2 and HCDR3 comprise amino acid sequences selected from the group consisting of:
(1) said HCDR1 comprising an amino acid sequence as set forth in SEQ ID NO: 11, said HCDR2 comprising an amino acid sequence as set forth in SEQ ID NO: 10, and said HCDR3 comprising an amino acid sequence as set forth in SEQ ID NO: 9; and
(2) said HCDR1 comprising an amino acid sequence as set forth in SEQ ID NO: 29, said HCDR2 comprising an amino acid sequence as set forth in SEQ ID NO: 28, and said HCDR3 comprising an amino acid sequence as set forth in SEQ ID NO: 27.

24. The isolated antigen-binding protein of any one of claims 1 to 23, wherein said second binding domain comprises H-FR1, the C-terminus of said H-FR1 is directly or indirectly linked to the N-terminus of said HCDR1, and said H-FR1 comprises an amino acid sequence as set forth in SEQ ID NO: 12 or SEQ ID NO: 30.

25. The isolated antigen-binding protein of any one of claims 1 to 24, wherein said second binding domain comprises H-FR2, said H-FR2 is between said HCDR1 and said HCDR2, and said H-FR2 comprises an amino acid sequence as set forth in SEQ ID NO: 13 or SEQ ID NO: 31.

26. The isolated antigen-binding protein of any one of claims 1 to 25, wherein said second binding domain comprises H-FR3, said H-FR3 is between said HCDR2 and said HCDR3, and said H-FR3 comprises an amino acid sequence as set forth in SEQ ID NO: 14 or SEQ ID NO: 32.

27. The isolated antigen-binding protein of any one of claims 1 to 26, wherein said second binding domain comprises H-FR4, the N-terminus of said H-FR4 is directly or indirectly linked to the C-terminus of said HCDR3, and said H-FR4 comprises an amino acid sequence as set forth in SEQ ID NO: 15.

28. The isolated antigen-binding protein of any one of claims 1 to 27, wherein said second binding domain comprises H-FR1, H-FR2, H-FR3 and H-FR4, and said H-FR1, H-FR2, H-FR3 and H-FR4 comprise amino acid sequences selected from the group consisting of:
(1) said H-FR1 comprising an amino acid sequence as set forth in SEQ ID NO: 12, said H-FR2 comprising an amino acid sequence as set forth in SEQ ID NO: 13, said H-FR3 comprising an amino acid sequence as set forth in SEQ ID NO: 14, and said H-FR4 comprising an amino acid sequence as set forth in SEQ ID NO: 15; and
(2) said H-FR1 comprising an amino acid sequence as set forth in SEQ ID NO: 30, said H-FR2 comprising an amino acid sequence as set forth in SEQ ID NO: 31, said H-FR3 comprising an amino acid sequence as set forth in SEQ ID NO: 32, and said H-FR4 comprising an amino acid sequence as set forth in SEQ ID NO: 15.

29. The isolated antigen-binding protein of any one of claims 1 to 28, wherein said second binding domain comprises VH, and said VH comprises an amino acid sequence as set forth in SEQ ID NO: 16 or SEQ ID NO: 33.

30. The isolated antigen-binding protein of any one of claims 1 to 29, wherein said second binding domain comprises an antibody heavy chain constant region.

31. The isolated antigen-binding protein of claim 30, wherein said antibody heavy chain constant region is derived from an IgG heavy chain constant region.

32. The isolated antigen-binding protein of any one of claims 30 to 31, wherein said antibody heavy chain constant region is derived from a human IgG heavy chain constant region.

33. The isolated antigen-binding protein of any one of claims 30 to 32, wherein said antibody heavy chain constant region is derived from a human IgG1 heavy chain constant region or a human IgG4 heavy chain constant region.

34. The isolated antigen-binding protein of any one of claims 30 to 33, wherein said antibody heavy chain constant region comprises an amino acid sequence as set forth in SEQ ID NO: 43.

35. The isolated antigen-binding protein of any one of claims 1 to 34, wherein said second binding domain comprises an antibody heavy chain, and said antibody heavy chain comprises an amino acid sequence as set forth in SEQ ID NO: 25 or SEQ ID NO: 41.

36. The isolated antigen-binding protein of any one of claims 1 to 35, wherein said second binding domain comprises at least one CDR in an antibody light chain variable region VL, and said VL comprises an amino acid sequence as set forth in SEQ ID NO: 24 or SEQ ID NO: 40.

37. The isolated antigen-binding protein of any one of claims 1 to 36, wherein said second binding domain comprises LCDR3, and said LCDR3 comprises an amino acid sequence as set forth in SEQ ID NO: 17 or SEQ ID NO: 34.

38. The isolated antigen-binding protein of any one of claims 1 to 37, wherein said second binding domain comprises LCDR2, and said LCDR2 comprises an amino acid sequence as set forth in SEQ ID NO: 18 (WAS) or SEQ ID NO: 35 (LAS).

39. The isolated antigen-binding protein of any one of claims 1 to 38, wherein said second binding domain comprises LCDR1, and said LCDR1 comprises an amino acid sequence as set forth in SEQ ID NO: 19 or SEQ ID NO: 36.

40. The isolated antigen-binding protein of any one of claims 1 to 39, wherein said second binding domain comprises LCDR1, LCDR2 and LCDR3, and said LCDR1, LCDR2 and LCDR3 comprise amino acid sequences selected from the group consisting of:
(1) said LCDR1 comprising an amino acid sequence as set forth in SEQ ID NO: 19, said LCDR2 comprising an amino acid sequence as set forth in SEQ ID NO: 18 (WAS), and said LCDR3 comprising an amino acid sequence as set forth in SEQ ID NO: 17; and
(2) said LCDR1 comprising an amino acid sequence as set forth in SEQ ID NO: 36, said LCDR2 comprising an amino acid sequence as set forth in SEQ ID NO: 35 (LAS), and said LCDR3 comprising an amino acid sequence as set forth in SEQ ID NO: 34.

41. The isolated antigen-binding protein of any one of claims 1 to 40, wherein said second binding domain comprises L-FR1, the C-terminus of said L-FR1 is directly or indirectly linked to the N-terminus of said L-CDR1, and said L-FR1 comprises an amino acid sequence as set forth in SEQ ID NO: 20 or SEQ ID NO: 37.

42. The isolated antigen-binding protein of any one of claims 1 to 41, wherein said second binding domain comprises L-FR2, said L-FR2 is between said LCDR1 and said LCDR2, and said L-FR2 comprises an amino acid sequence as set forth in SEQ ID NO: 21 or SEQ ID NO: 38.

43. The isolated antigen-binding protein of any one of claims 1 to 42, wherein said second binding domain comprises L-FR3, said L-FR3 is between said LCDR2 and said LCDR3, and said L-FR3 comprises an amino acid sequence as set forth in SEQ ID NO: 22 or SEQ ID NO: 39.

44. The isolated antigen-binding protein of any one of claims 1 to 43, wherein said second binding domain comprises L-FR4, the N-terminus of said L-FR4 is directly or indirectly linked to the C-terminus of said LCDR3, and said L-FR4 comprises an amino acid sequence as set forth in SEQ ID NO: 23.

45. The isolated antigen-binding protein of any one of claims 1 to 44, wherein said second binding domain comprises L-FR1, L-FR2, L-FR3 and L-FR4, and said L-FR1, L-FR2, L-FR3 and L-FR4 comprise amino acid sequences selected from the group consisting of:
(1) said L-FR1 comprising an amino acid sequence as set forth in SEQ ID NO: 20, said L-FR2 comprising an amino acid sequence as set forth in SEQ ID NO: 21, said L-FR3 comprising an amino acid sequence as set forth in SEQ ID NO: 22, and said L-FR4 comprising an amino acid sequence as set forth in SEQ ID NO: 23; and
(2) said L-FR1 comprising an amino acid sequence as set forth in SEQ ID NO: 37, said L-FR2 comprising an amino acid sequence as set forth in SEQ ID NO: 39, said L-FR3 comprising an amino acid sequence as set forth in SEQ ID NO: 39, and said L-FR4 comprising an amino acid sequence as set forth in SEQ ID NO: 23.

46. The isolated antigen-binding protein of any one of claims 1 to 45, wherein said second binding domain comprises VL, and said VL comprises an amino acid sequence as set forth in SEQ ID NO: 24 or SEQ ID NO: 40.

47. The isolated antigen-binding protein of any one of claims 1 to 46, wherein said second binding domain comprises an antibody light chain constant region.

48. The isolated antigen-binding protein of claim 47, wherein said antibody light chain constant region is derived from an Igκ constant region.

49. The isolated antigen-binding protein of any one of claims 47 to 48, wherein said antibody light chain constant region is derived from a human Igκ constant region.

50. The isolated antigen-binding protein of any one of claims 47 to 49, wherein said antibody light chain constant region comprises an amino acid sequence as set forth in SEQ ID NO: 44.

51. The isolated antigen-binding protein of any one of claims 1 to 50, wherein said second binding domain comprises an antibody light chain, and said antibody light chain comprises an amino acid sequence as set forth in SEQ ID NO: 26 or SEQ ID NO: 42.

52. The isolated antigen-binding protein of any one of claims 1 to 51, wherein said second binding domain comprises VH and VL, and said VH and VL comprise amino acid sequences selected from the group consisting of:
(1) said VH comprising an amino acid sequence as set forth in SEQ ID NO: 16, and said VL comprising an amino acid sequence as set forth in SEQ ID NO: 24; and
(2) said VH comprising an amino acid sequence as set forth in SEQ ID NO: 33, and said VL comprising an amino acid sequence as set forth in SEQ ID NO: 40.

53. The isolated antigen-binding protein of any one of claims 1 to 52, wherein said second binding domain comprises an antibody heavy chain and an antibody light chain, and said antibody heavy chain and antibody light chain comprise amino acid sequences selected from the group consisting of:
(1) said antibody heavy chain comprising an amino acid sequence as set forth in SEQ ID NO: 25, and said antibody light chain comprising an amino acid sequence as set forth in SEQ ID NO: 26; and
(2) said antibody heavy chain comprising an amino acid sequence as set forth in SEQ ID NO: 41, and said antibody light chain comprising an amino acid sequence as set forth in SEQ ID NO: 42.

54. The isolated antigen-binding protein of any one of claims 1 to 53, wherein said first binding domain and said second binding domain are directly or indirectly linked.

55. The isolated antigen-binding protein of any one of claims 1 to 54, wherein the C-terminus of said first binding domain is linked to the N-terminus of said second binding domain.

56. The isolated antigen-binding protein of any one of claims 1 to 55, wherein the C-terminus of said first binding domain is linked to the N-terminus of a heavy chain variable region of said second binding domain.

57. The isolated antigen-binding protein of any one of claims 1 to 56, wherein said first binding domain is linked to said second binding domain by a linker.

58. The isolated antigen-binding protein of claim 57, wherein said linker comprises a peptide linker.

59. The isolated antigen-binding protein of any one of claims 57 to 58, wherein said linker comprises an amino acid sequence of (GGGGS) n, where n is any positive integer from 0 to 10.

60. The isolated antigen-binding protein of any one of claims 57 to 59, wherein said linker comprises an amino acid sequence as set forth in SEQ ID NO: 47.

61. The isolated antigen-binding protein of any one of claims 1 to 60, comprising two of said first binding domains.

62. The isolated antigen-binding protein of claim 61, wherein said two first binding domains are directly or indirectly linked to the N-terminus of said second binding domain respectively.

63. The isolated antigen-binding protein of any one of claims 61 to 62, wherein said two first binding domains are directly or indirectly linked to the N-termini of two heavy chains of said second binding domain respectively.

64. The isolated antigen-binding protein of any one of claims 1 to 63, comprising a first polypeptide chain and a second polypeptide chain, wherein said first polypeptide chain comprises heavy chains of said first binding domain and said second binding domain.

65. The isolated antigen-binding protein of claim 64, wherein the N-termini of heavy chains of said first binding domain and said second binding domain are directly or indirectly linked.

66. The isolated antigen-binding protein of any one of claims 64 to 65, wherein the N-termini of heavy chains of said first binding domain and said second binding domain are linked by a linker.

67. The isolated antigen-binding protein of any one of claims 64 to 66, wherein said first polypeptide chain comprises an amino acid sequence as set forth in SEQ ID NO: 45 or SEQ ID NO: 46.

68. The isolated antigen-binding protein of any one of claims 64 to 67, wherein said second polypeptide chain comprises a light chain of said second binding domain.

69. The isolated antigen-binding protein of any one of claims 64 to 68, wherein said second polypeptide chain comprises an amino acid sequence as set forth in SEQ ID NO: 26 or SEQ ID NO: 42.

70. The isolated antigen-binding protein of any one of claims 64 to 69, comprising two of said first polypeptide chains and two of said second polypeptide chains.

71. A pharmaceutical combination, comprising an antigen-binding protein capable of binding to CD39 and an antigen-binding protein capable of binding to CD73.

72. The pharmaceutical combination of claim 71, wherein said antigen-binding protein capable of binding to CD39 has one or more of the following properties:
(1) capability of binding to human CD39 at an EC50 less than about 0.5 µg/mL according to an FACS detection method;
(2) capability of binding to cynomolgus (cyno) CD39 at an EC50 less than about 0.7 µg/mL according to an FACS detection method;
(3) capability of binding to human CD39 at a KD less than about 1.3×10⁻⁸ M according to a Biacore detection method;
(4) capability of binding to cynomolgus (cyno) CD39 at a KD less than about 1.1×10⁻⁷ M according to a Biacore detection method;
(5) capability of inhibiting ATPase activity in CD39 expressing cells at an EC50 less than about 0.8 µg/mL according to an ATPase activity analysis; and
(6) capability of inhibiting the function of CD39 of tumor cells.

73. The pharmaceutical combination of any one of claims 71 to 72, wherein said antigen-binding protein capable of binding to CD39 comprises at least one CDR in a heavy chain variable region VH, and said VH comprises an amino acid sequence as set forth in SEQ ID NO: 8.

74. The pharmaceutical combination of any one of claims 71 to 73, wherein said antigen-binding protein capable of binding to CD39 comprises HCDR3, and said HCDR3 comprises an amino acid sequence as set forth in SEQ ID NO: 1.

75. The pharmaceutical combination of any one of claims 71 to 74, wherein said antigen-binding protein capable of binding to CD39 comprises HCDR2, and said HCDR2 comprises an amino acid sequence as set forth in SEQ ID NO: 2.

76. The pharmaceutical combination of any one of claims 71 to 75, wherein said antigen-binding protein capable of binding to CD39 comprises HCDR1, and said HCDR1 comprises an amino acid sequence as set forth in SEQ ID NO: 3.

77. The pharmaceutical combination of any one of claims 71 to 76, wherein said antigen-binding protein capable of binding to CD39 comprises VH, and said VH comprises an amino acid sequence as set forth in SEQ ID NO: 8.

78. The pharmaceutical combination of any one of claims 71 to 77, wherein said antigen-binding protein capable of binding to CD39 is VHH.

79. The pharmaceutical combination of any one of claims 71 to 78, wherein said antigen-binding protein capable of binding to CD73 has one or more of the following properties:
(1) capability of specifically binding to CD73 or a functional active fragment thereof;
(2) capability of inhibiting the enzymatic activity of CD73;
(3) capability of inducing endocytosis of CD73 on the cell surface; and
(4) capability of inhibiting growth and/or proliferation of tumor cells.

80. The pharmaceutical combination of any one of claims 71 to 79, wherein said antigen-binding protein capable of binding to CD73 comprises at least one CDR in an antibody heavy chain variable region VH, and said VH comprises an amino acid sequence as set forth in SEQ ID NO: 16 or SEQ ID NO: 33.

81. The pharmaceutical combination of any one of claims 71 to 80, wherein said antigen-binding protein capable of binding to CD73 comprises HCDR3, and said HCDR3 comprises an amino acid sequence as set forth in SEQ ID NO: 9 or SEQ ID NO: 27.

82. The pharmaceutical combination of any one of claims 71 to 81, wherein said antigen-binding protein capable of binding to CD73 comprises HCDR2, and said HCDR2 comprises an amino acid sequence as set forth in SEQ ID NO: 10 or SEQ ID NO: 28.

83. The pharmaceutical combination of any one of claims 71 to 82, wherein said antigen-binding protein capable of binding to CD73 comprises HCDR1, and said HCDR1 comprises an amino acid sequence as set forth in SEQ ID NO: 11 or SEQ ID NO: 29.

84. The pharmaceutical combination of any one of claims 71 to 83, wherein said antigen-binding protein capable of binding to CD73 comprises VH, and said VH comprises an amino acid sequence as set forth in SEQ ID NO: 16 or SEQ ID NO: 33.

85. The pharmaceutical combination of any one of claims 71 to 84, wherein said antigen-binding protein capable of binding to CD73 comprises an antibody heavy chain constant region.

86. The pharmaceutical combination of claim 85, wherein said antibody heavy chain constant region is derived from an IgG heavy chain constant region.

87. The pharmaceutical combination of any one of claims 85 to 86, wherein said antibody heavy chain constant region is derived from a human IgG heavy chain constant region.

88. The pharmaceutical combination of any one of claims 85 to 87, wherein said antibody heavy chain constant region is derived from a human IgG1 heavy chain constant region or a human IgG4 heavy chain constant region.

89. The pharmaceutical combination of any one of claims 85 to 88, wherein said antibody heavy chain constant region comprises an amino acid sequence as set forth in SEQ ID NO: 43.

90. The pharmaceutical combination of any one of claims 71 to 89, wherein said antigen-binding protein capable of binding to CD73 comprises an antibody heavy chain, and said antibody heavy chain comprises an amino acid sequence as set forth in SEQ ID NO: 25 or SEQ ID NO: 41.

91. The pharmaceutical combination of any one of claims 71 to 90, wherein said antigen-binding protein capable of binding to CD73 comprises at least one CDR in an antibody light chain variable region VL, and said VL comprises an amino acid sequence as set forth in SEQ ID NO: 24 or SEQ ID NO: 40.

92. The pharmaceutical combination of any one of claims 71 to 91, wherein said antigen-binding protein capable of binding to CD73 comprises LCDR3, and said LCDR3 comprises an amino acid sequence as set forth in SEQ ID NO: 17 or SEQ ID NO: 34.

93. The pharmaceutical combination of any one of claims 71 to 92, wherein said antigen-binding protein capable of binding to CD73 comprises LCDR2, and said LCDR2 comprises an amino acid sequence as set forth in SEQ ID NO: 18 (WAS) or SEQ ID NO: 35 (LAS).

94. The pharmaceutical combination of any one of claims 71 to 93, wherein said antigen-binding protein capable of binding to CD73 comprises LCDR1, and said LCDR1 comprises an amino acid sequence as set forth in SEQ ID NO: 19 or SEQ ID NO: 36.

95. The pharmaceutical combination of any one of claims 71 to 94, wherein said antigen-binding protein capable of binding to CD73 comprises VL, and said VL comprises an amino acid sequence as set forth in SEQ ID NO: 24 or SEQ ID NO: 40.

96. The pharmaceutical combination of any one of claims 71 to 95, wherein said antigen-binding protein capable of binding to CD73 comprises an antibody light chain constant region.

97. The pharmaceutical combination of claim 96, wherein said antibody light chain constant region is derived from a human Igκ constant region.

98. The pharmaceutical combination of any one of claims 96 to 97, wherein said antibody light chain constant region comprises an amino acid sequence as set forth in SEQ ID NO: 44.

99. The pharmaceutical combination of any one of claims 71 to 98, wherein said antigen-binding protein capable of binding to CD73 comprises an antibody light chain, and said antibody light chain comprises an amino acid sequence as set forth in SEQ ID NO: 26 or SEQ ID NO: 42.

100. The pharmaceutical combination of any one of claims 71 to 99, wherein said antigen-binding protein capable of binding to CD73 comprises VH and VL, and said VH and VL comprise amino acid sequences selected from the group consisting of:
(1) said VH comprising an amino acid sequence as set forth in SEQ ID NO: 16, and said VL comprising an amino acid sequence as set forth in SEQ ID NO: 24; and
(2) said VH comprising an amino acid sequence as set forth in SEQ ID NO: 33, and said VL comprising an amino acid sequence as set forth in SEQ ID NO: 40.

101. The pharmaceutical combination of any one of claims 71 to 100, wherein said antigen-binding protein capable of binding to CD73 comprises an antibody heavy chain and an antibody light chain, and said antibody heavy chain and antibody light chain comprise amino acid sequences selected from the group consisting of:
(1) said antibody heavy chain comprising an amino acid sequence as set forth in SEQ ID NO: 25, and said antibody light chain comprising an amino acid sequence as set forth in SEQ ID NO: 26; and
(2) said antibody heavy chain comprising an amino acid sequence as set forth in SEQ ID NO: 41, and said antibody light chain comprising an amino acid sequence as set forth in SEQ ID NO: 42.

102. One or more isolated nucleic acid molecules, encoding the isolated antigen-binding protein of any one of claims 1 to 70.

103. A vector, comprising the nucleic acid molecule of claim 102.

104. A cell, comprising the nucleic acid molecule of claim 102 or the vector of claim 103.

105. A pharmaceutical composition, comprising the isolated antigen-binding protein of any one of claims 1 to 70, the pharmaceutical combination of any one of claims 71 to 101, the nucleic acid molecule of claim 102, the vector of claim 103, and/or the cell of claim 1 to 4, and optionally a pharmaceutically acceptable carrier.

106. A method for preparing the isolated antigen-binding protein of any one of claims 1 to 70, comprising: culturing the cell of claim 104 under a condition that allows expression of the isolated antigen-binding protein of any one of claims 1 to 70.

107. Use of the isolated antigen-binding protein of any one of claims 1 to 70, the pharmaceutical combination of any one of claims 71 to 101, the nucleic acid molecule of claim 102, the vector of claim 103, the cell of claim 104, and/or the pharmaceutical composition of claim 105 in preparation of a drug, wherein said drug is used for preventing and/or treating diseases and/or disorders.

108. The use of claim 107, wherein said diseases and/or disorders are mediated by CD39 and/or CD73.

109. The use of claims 107 to 108, wherein said diseases and/or disorders comprise tumors.

110. The use of claim 109, wherein said tumors comprise solid tumors and/or hematologic tumors.

111. The use of any one of claims 107 to 110, wherein said diseases and/or disorders comprise pancreatic cancer and/or colorectal cancer.

112. A method for detecting CD39 and/or CD73 in a sample, comprising administration of the isolated antigen-binding protein of any one of claims 1 to 70, the pharmaceutical combination of any one of claims 71 to 101, the nucleic acid molecule of claim 102, the vector of claim 103, the cell of claim 104, and/or the pharmaceutical composition of claim 105.

113. The method of claim 112, which is an in vitro and/or ex vivo method.

114. Use of the isolated antigen-binding protein of any one of claims 1 to 70, the pharmaceutical combination of any one of claims 71 to 101, the nucleic acid molecule of claim 102, the vector of claim 103, the cell of claim 104, and/or the pharmaceutical composition of claim 105 in preparation of a kit, wherein said kit is used for detecting presence of CD39 and/or CD73 in a sample.
